# FASCICULE DE BREVET EUROPEEN

(11) **EP 2 890 555 B1**
(45) Date de publication et mention de la délivrance du brevet: **28.09.2016**
(21) Numéro de dépôt: 13759685.4
(22) Date de dépôt: 27.08.2013
(51) Int. Cl.: B32B 5/18, C08H 8/00, C08J 9/28, C08G 8/20, C08G 14/04, C08L 61/12, F16L 59/00

(54) **MONOLITHES POREUX CELLULAIRES A BASE DE TANNINS CONDENSES**
PORÖSE ZELLULÄRE MONOLITHEN MIT KONDENSIERTEN TANNINEN
CELLULAR POROUS MONOLITHS CONTAINING CONDENSED TANNINS

(30) Priorité: 28.08.2012 FR 1258032
(43) Date de publication de la demande: 08.07.2015
(73) Titulaire: Université de Lorraine, 54052 Nancy Cedex (FR); Centre National de la Recherche Scientifique (CNRS), 75016 Paris (FR)
(72) Inventeur: CELZARD, Alain, F-88000 Epinal (FR); SZCZUREK, Andrzej, F-54600 Villers-les-Nancy (FR); FIERRO, Vanessa, F-88000 Epinal (FR); PIZZI, Antonio, F-88000 Chantraine (FR)
(74) Mandataire: Pontet Allano & Associes
(86) Numéro de dépôt international: PCT/EP2013/067716
(87) Numéro de publication internationale: WO 2014/033124

(56) Documents cités:
- US-A1- 2007 049 887
- BUCHMEISER ET AL: "Polymeric monolithic materials: Syntheses, properties, functionalization and applications", POLYMER, ELSEVIER SCIENCE PUBLISHERS B.V, GB, vol. 48, no. 8, 29 mars 2007 (2007-03-29), pages 2187-2198, XP022008014, ISSN: 0032-3861, DOI: 10.1016/J.POLYMER.2007.02.045
- ZHANG S ET AL: "Stability of high internal phase emulsions with sole cationic surfactant and its tailoring morphology of porous polymers based on the emulsions", POLYMER, ELSEVIER SCIENCE PUBLISHERS B.V, GB, vol. 50, no. 7, 20 mars 2009 (2009-03-20), pages 1723-1731, XP025976819, ISSN: 0032-3861, DOI: 10.1016/J.POLYMER.2008.11.004 [extrait le 2008-11-12]
- TONDI G ET AL: "Tannin-based carbon foams", CARBON, ELSEVIER, OXFORD, GB, vol. 47, no. 6, 1 mai 2009 (2009-05-01), pages 1480-1492, XP026077032, ISSN: 0008-6223, DOI: 10.1016/J.CARBON.2009.01.041 [extrait le 2009-02-03]

## Description

La présente invention a pour objet un procédé de production de monolithes poreux cellulaires d'origine naturelle à base de tannins condensés, les monolithes obtenus par ce procédé et leurs applications ainsi que l'émulsion et la mousse liquide permettant leur fabrication.

Les matériaux " polyHIPE " (pour Polymerised High Internal Phase Emulsion) proposés pour la première fois en 1982 (EP 0060138) sont obtenus par polymérisation d'une émulsion dénommée HIPE (pour High Internal Phase Emulsion) composée d'une part, d'une phase externe ou dispersante qui est essentiellement constituée de monomères polymérisables et d'un agent tensioactif en solution dans un solvant, et d'autre part, d'une phase interne ou dispersée qui représente typiquement 74% ou plus du volume total de l'émulsion et qui est essentiellement constituée d'un solvant non miscible aux monomères polymérisables ou au solvant de la phase dispersante. Après polymérisation et élimination du solvant de la phase dispersée, on obtient des matériaux à cellules ouvertes dont les cellules correspondent à l'empreinte des bulles formées par ce solvant au cours de la préparation de l'émulsion et sont interconnectées par des ouvertures de plus petite taille qu'elles, communément désignées sous le terme de pores. Compte tenu de leurs caractéristiques, les matériaux polyHIPE sont l'objet d'un intérêt croissant et leur utilisation a été proposée dans de nombreux domaines parmi lesquels on peut citer la fabrication d'articles absorbants jetables, d'articles d'isolation thermique, acoustique, électrique ou mécanique, de membranes, de filtres ou encore de supports pour les encres, les colorants et les catalyseurs.

Les mousses sont des matériaux complexes constitués par la dispersion d'un gaz dans un milieu condensé. Selon le type de milieu condensé, les mousses peuvent être liquides ou solides.

La formation des mousses est un processus que l'on observe régulièrement dans la nature quand en particulier des gaz sont mélangés mécaniquement dans un liquide.

Les mousses liquides sont des matériaux que l'on utilise quotidiennement: les mousses tensioactives, la mousse à raser, la mousse du lait, du cappuccino ou de la bière.

Les mousses solides sont plus difficiles à trouver dans la nature. En général, elles sont produites à partir du dégagement d'un gaz dans un milieu liquide à haute viscosité qui durcit pendant que le gaz s'échappe.

Les mousses solides sont une classe de matériaux caractérisés généralement par leur légèreté et leur structure alvéolaire qui garantissent des solutions intéressantes d'un point de vue applicatif. Elles peuvent être classées selon le type des cellules. Les mousses à cellules ouvertes présentent des cellules très interconnectées, et leur structure est de ce fait très perméable et légère. Les mousses à cellules fermées ont une résistance beaucoup plus grande que les précédentes parce que les parois ne sont pas trouées et donc peuvent soutenir des efforts de compression plus importants.

Une autre classification des mousses solides est basée sur les propriétés physiques:
- Les mousses élastiques ont la propriété d'être déformables tout en reprenant leur forme d'origine lorsque la contrainte qu'on leur applique disparait. Le marché de ces mousses est dominé par les mousses polyuréthane mais les latex et les mousses EVA sont aussi très utilisés dans des secteurs spécifiques comme les matelas et les accessoires sportifs.
- Les mousses rigides au contraire sont des matériaux qui ne se déforment pas et qui trouvent comme principales applications, l'isolation thermique et acoustique des bâtiments. Les mousses rigides sont parfois utilisées comme anti-choc dans les automobiles pour leur capacité d'absorber les contraintes et l'énergie mécanique et pour leur légèreté. Les mousses rigides les plus vendues sont les polyuréthanes et les phénoliques.

La très grande majorité des matériaux polyHIPE sont obtenus à partir de polymères issus de ressources pétrolières peu respectueuses de l'environnement et dont la raréfaction entraîne une hausse des coûts de production. Aussi, pour diminuer les coûts et se placer dans le cadre d'alternatives plus respectueuses de l'environnement à ces produits synthétiques dérivés de la pétrochimie et dont la production nécessite de lourdes dépenses énergétiques, on s'est tourné vers les matériaux « verts ».

En ce qui concerne les mousses élastiques, les produits commerciaux sont majoritairement constitués de polyuréthanes mais il existe des alternatives naturelles, notamment les latex qui se développent de plus en plus sur le marché. En revanche, dans le cas des mousses rigides, pratiquement aucun produit naturel n'est proposé en substitution des produits synthétiques.

Les matériaux « verts », notamment dérivés de la biomasse, devraient progressivement remplacer leurs homologues synthétiques plus chers et moins respectueux de l'environnement. Par biomasse, on entend tous les produits ligno-cellulosiques issus de l'agriculture et de la sylviculture (pailles, noyaux et écorces de fruits, bois et tous autres résidus végétaux), mais également leurs dérivés après séparation par des opérations chimiques, thermochimiques ou mécanochimiques (lignine, cellulose, tannins, et tous autres composés furaniques et phénoliques). Le caractère multifonctionnel des matériaux dérivés de la chimie verte permet une extraordinaire variété d'applications dans les domaines de l'énergie et de l'environnement.

Jusqu'à ce jour peu de travaux ont été réalisés et les seuls matériaux de type polyHIPE fabriqués à partir de matériau « vert » ont été élaborés à partir de liqueur noire Kraft, sous-produit de l'industrie papetière (http://www.theses.fr/2011BOR14435). Cette liqueur, lourdement chargée en minéraux divers, est difficile à utiliser puisque la moindre variation de pH entraîne la précipitation de la lignine. D'autre part, les matériaux sont extrêmement impurs, ce qui peut être préjudiciable pour certaines applications.

Aussi les inventeurs se sont posé le problème de trouver un autre matériau issu de la biomasse qui ne présenterait pas ces inconvénients.

Bien connu pour le traitement du cuir, les tannins sont des composants polyphénoliques utilisés par les plantes pour se défendre des insectes et des champignons. Ces produits se trouvent dans toutes les plantes en différents pourcentages. Les écorces des arbres en général en contiennent la quantité la plus significative, mais le tannin est présent dans le cytoplasme de toutes les cellules végétales. Les différents bois stockent les tannins dans différentes zones de la plante : le pin *(Pinus radiata),* le chêne (*Quercus robur*) et le mimosa (*Acacia mearnsii ou mollissima*) contiennent la majorité de leurs tannins dans l'écorce ; le gambier (*Uncaria Gambir*) dans les feuilles tandis que le châtaignier (*Castanea sativa*) et le quebracho (*Schinopsis balansae*) stockent leurs tannins dans toute la structure. Les tannins végétaux peuvent se combiner aux protéines pour donner des complexes solubles ou insolubles. Ils possèdent malgré les différences de leurs constitutions un ensemble de caractères communs :
- Ils précipitent les protéines de leur solution, en particulier la gélatine.
- Ils donnent avec les sels des métaux lourds des laques de couleurs variées.
- Ils précipitent avec les matières colorantes cationiques.
- Ils sont plus ou moins solubles dans l'eau et leurs solutions sont toujours acides.
- Ils sont amorphes et sans point de fusion précis.

Du point de vue de la composition chimique, on distingue deux grandes familles de tannins : les tannins hydrolysables et les tannins condensés ou flavonoïdes.

Les tannins hydrolysables sont constitués de produits phénoliques simples : ce sont des esters d'acide gallique et de ses dimères (acide digallique, acide ellagique) et de monosaccharides (surtout le glucose). Les tannins hydrolysables sont souvent divisés en gallotannins, conduisant à de l'acide gallique après hydrolyse, ou en ellagitannins libérant de l'acide ellagique après hydrolyse. Ils ont déjà été utilisés comme substituts partiels du phénol dans la fabrication de résines phénol-formaldéhyde, néanmoins leur utilisation reste des plus limitées dans le domaine des colles du fait de leur basse réactivité avec le formaldéhyde. En revanche, les tannins de châtaignier et de tara sont très utilisés dans l'industrie du tannage.

A l'opposé des tannins hydrolysables, les tannins condensés ne sont pas décomposables par hydrolyse. Au contraire, soumis à un chauffage en milieu acide ils se polymérisent progressivement et forment des pigments anthocyaniques, amorphes, de coloration rouge, ou des produits bruns jaunes, insolubles, de poids moléculaire élevé, appelés phlobaphènes. Lors de leur pyrolyse, les tannins condensés fournissent du pyrocatéchol.

Les tannins condensés sont constitués d'unités flavonoïdes classifiées en quatre entités (Porter, L.J. : The flavonoids. J.B. Harborne, Ed., Chapman and Hall, London, 1988)
- les tannins de type prodelphinidine possédant un anneau A de type phloroglucinol et un anneau B de type pyrogallol (l'élément de base est la gallocatéchine, figure A), 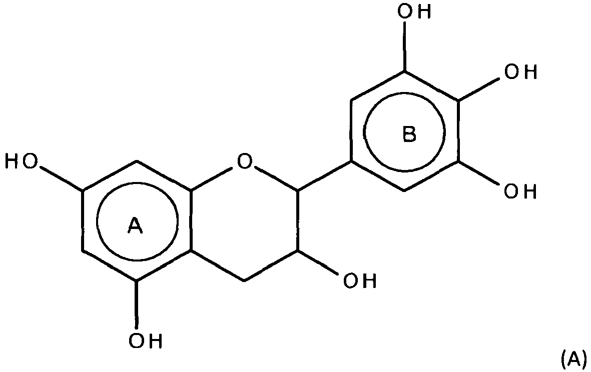
- les tannins de type procyanidine possédant un anneau A de type phloroglucinol et un anneau B de type catéchol (l'élément de base est la catéchine, figure B) 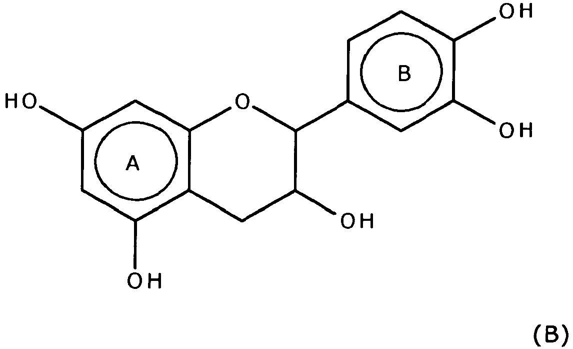
- les tannins de type prorobinetinidine possédant un anneau A de type résorcinol et un anneau B de type pyrogallol (l'élément de base est le robinetinidol, figure C), et 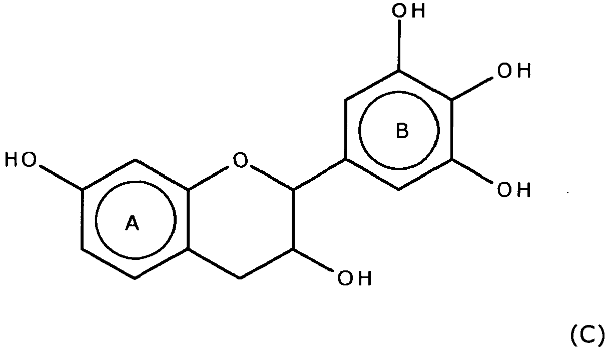
- les tannins de type profisetinidine possédant un anneau A de type résorcinol et un anneau B de type catéchol (l'élément de base est le fisetinidol, figure D).

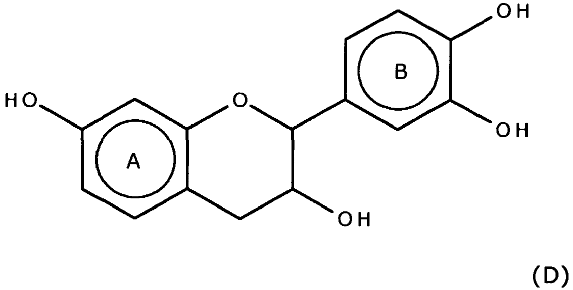

Les unités de tannins condensés sont généralement liées par des liaisons 4-6 et 4-8. Les tannins condensés ont une répétition de 2 à 8 unités flavonoïdes.

Mélangés à de l'eau, à un durcisseur et à un agent moussant, les tannins produisent des mousses rigides extrêmement légères. Leurs propriétés remarquables, similaires et même supérieures aux mousses phénoliques commerciales actuellement utilisées en aéronautique et marine, combinent résistance mécanique, isolation thermique, ininflammabilité et infusibilité.

Placés dans d'autres conditions, les tannins polymérisent pour donner des gels rigides, plus ou moins élastiques. A densité équivalente à celle des mousses rigides, ce sont des matériaux dont la porosité est 1000 à 10 000 fois plus étroite qui sont obtenus. On ne parle alors plus d'isolants mais potentiellement de « super isolants » thermiques. Les concurrents directs de tels solides ultra-légers sont les aérogels de silice, très onéreux et issus d'une chimie toxique. Les aérogels de tannins sont plus légers, moins chers, non irritants et opaques, ce qui est susceptible de les rendre encore meilleurs en ne transmettant pas ou très peu l'infrarouge.

La pyrolyse de ces deux familles de matériaux (mousses et gels), conduit à leurs homologues en carbone vitreux. La structure poreuse de départ est conservée, mais la résistance mécanique s'est améliorée avec le traitement thermique, en même temps que la résistance aux chocs thermiques et l'inertie chimique.

Une autre propriété d'intérêt est apparue : la conductivité électrique. Non contents de conserver les applications de leurs précurseurs organiques (composites sandwich, isolation thermique et phonique, absorption des chocs, filtration de fluides corrosifs ou de métaux fondus), les mousses de carbone dérivées peuvent aussi être utilisées comme électrodes poreuses, pour le blindage électromagnétique, la catalyse hétérogène, l'adsorption, ... Les aérogels de carbone ex tannins ont par ailleurs d'excellentes performances en tant qu'électrode de supercondensateurs. Ces dispositifs, qui servent de puissance électrique d'appoint dans les tramways, TGV et autres véhicules électriques ou hybrides, sont appelés à se développer et exigent des caractéristiques d'inertie chimique et de porosité que les gels de carbone sont en mesure d'apporter. Environ 5 fois moins chers que leurs homologues dérivés du résorcinol, les aérogels de carbone ex tannins sont des concurrents sérieux pour le stockage d'énergie électrochimique.

Les inventeurs ont déjà décrit des mousses à base de tannin préparées de façon totalement différente, par moussage physique et/ou chimique, c'est-à-dire que la mousse est formée par expansion et/ou production d'un gaz dans la formulation (Tondi G. et al. Carbon (2009), 47, no 6, pages 1480-1492 ; Bioresource Technology (2009), 100, no 21, pages 5162-5169 ; Zhao W. et al. Materials Chemistry and Physics (2010), 122, 175-182 ; Zhao W. et al. Materials Chemistry and Physics (2010), 123, 210-217 ; Basso M.C. et al. Advanced Materials Letters (2011), 2, 378-382 ; Li X. et al. Maderas Ciencia y Tecnologia (2012), 14, 257-265 ; Li X. et al. Carbon (2012), 50, 2026 - 2036 ; Lacoste C. et al. Industrial Crops and Products (2013), 43, 245-250) et n'est pas de type polyHIPE.

Il serait donc souhaitable de disposer d'un procédé de préparation de matériaux qui soient soit des polyHIPE, soit des mousses, soit des produits hybrides entre polyHIPE et mousses et qui présentent une résistance mécanique élevée ou, à tout le moins, suffisamment élevée pour que leur utilisation dans toutes les applications qui ont été proposées pour ce type de matériaux issus de ressources pétrolières puisse être réellement envisagée, et qui mette en oeuvre des matériaux issus de la biomasse, peu coûteux à produire.

Aussi un premier objet de la présente invention est un procédé de production de matériaux monolithiques poreux à base de tannins condensés, ledit procédé comprenant les étapes suivantes :
a. obtenir une première phase liquide, ladite phase liquide étant une solution aqueuse de tannins condensés,
b. obtenir une seconde phase, ladite seconde phase étant soit une huile ou un solvant volatil non miscible à l'eau, soit de l'air, soit un mélange d'huile et d'air ou d'un solvant volatil non miscible à l'eau et d'air et ladite seconde phase n'étant pas miscible dans la première phase, et l'une au moins desdites phases comprenant un tensioactif,
c. disperser ladite seconde phase dans ladite première phase liquide, en présence d'un durcisseur,
d. mélanger lesdites phases sous agitation jusqu'à obtention :
   i. d'une émulsion homogène et stable lorsque la seconde phase est une huile ou un solvant volatil non miscible à l'eau, ou
   ii. d'un mélange macroscopiquement homogène mais intermédiaire entre une émulsion et une mousse lorsque la seconde phase est un mélange d'huile et d'air ou d'un solvant volatil non miscible à l'eau et d'air, ou
   iii. d'une mousse lorsque la seconde phase est de l'air, et
e. soit,
   i. réaliser la polymérisation de l'émulsion ou de l'intermédiaire émulsion-mousse obtenus à l'étape d.i. ou à l'étape d.ii jusqu'à l'obtention d'un solide, laver si nécessaire et sécher ledit solide, soit,
   ii. réaliser la polymérisation et sécher ladite mousse obtenue à l'étape d.iii.

Conformément à l'invention, l'étape de lavage en e.i. n'est nécessaire que si l'on utilise, comme seconde phase, de l'huile ou un mélange d'huile et d'air.

Conformément à l'invention, le tensioactif peut être présent soit dans la phase liquide aqueuse, soit dans la seconde phase lorsqu'elle contient de l'huile ou un solvant volatil non miscible à l'eau, soit dans la phase liquide aqueuse et dans la seconde phase lorsqu'elle contient de l'huile ou un solvant volatil non miscible à l'eau.

Conformément à l'invention, l'huile peut être de toute origine, animale, végétale ou synthétique. Elle est avantageusement d'origine végétale et avantageusement choisie parmi les huiles de palme, de soja, de colza, de tournesol, d'arachide, de ricin, de lin et d'olive. L'huile de tournesol est particulièrement appréciée. La quantité d'huile a une influence directe sur la porosité du matériau puisque la taille et le nombre des pores dépendent de la quantité d'huile mise en oeuvre. L'homme du métier saura définir à la lumière de ses connaissances et par des tests de routine la quantité d'huile nécessaire à l'obtention d'un matériau de porosité souhaitée.

Conformément à l'invention, le solvant volatil peut être n'importe quel solvant volatil connu de l'homme du métier du moment qu'il n'est pas miscible à l'eau (immiscible à l'eau), par exemple le cyclohexane ou l'heptane. Le terme « solvant volatil non miscible dans l'eau » recouvre également des mélanges de solvants volatils non miscibles dans l'eau mais miscibles entre eux.

Dans un mode de réalisation avantageux du procédé de l'invention :
A. soit la première phase liquide est une phase aqueuse de tannin condensé et la seconde phase une huile végétale ou un solvant volatil non miscible dans l'eau et on obtient, après durcissement de la phase aqueuse, extraction de l'huile lorsque la seconde phase est de l'huile végétale, puis séchage, un polyHIPE,
B. soit la première phase liquide est une phase aqueuse de tannin condensé et la seconde phase de l'air et on obtient, après durcissement de la phase aqueuse puis séchage, une mousse rigide,
C. soit la première phase liquide est une phase aqueuse de tannin condensé et la seconde phase un mélange d'huile végétale et d'air ou d'un solvant volatil non miscible dans l'eau et d'air et on obtient, après durcissement de la phase aqueuse, extraction de l'huile lorsque la seconde phase est de l'huile végétale, puis séchage, un matériau aéré « de type polyHIPE ».

Ainsi, conformément à l'invention, l'étape d'extraction d'huile dans les modes de réalisation A ou C n'est nécessaire que si l'on utilise, comme seconde phase, de l'huile ou un mélange d'huile et d'air. Cette étape n'est pas nécessaire lorsqu'on utilise un solvant volatil non miscible à l'eau ou un mélange d'un solvant volatil non miscible à l'eau et d'air.

Dans le mode de réalisation A, la porosité du matériau est obtenue par l'extraction de l'huile végétale du matériau durci ou par simple évaporation du solvant volatil non miscible à l'eau du matériau durci. Dans le mode de réalisation B, la porosité du matériau est obtenue par les bulles d'air incorporées dans le matériau durci. Dans le mode de réalisation C, la porosité du matériau est obtenue à la fois par l'extraction de l'huile végétale du matériau durci et par l'incorporation de l'air ou par l'évaporation du solvant volatil non miscible à l'eau du matériau durci et par l'incorporation de l'air ; au sens de la présente invention, on entend par matériau aéré « de type polyHIPE », un matériau intermédiaire entre une mousse rigide et un polyHIPE.

Dans les modes de réalisation A et B les inventeurs ont donc mis au point un procédé d'émulsion, notamment d'une émulsion huile végétale dans l'eau ou d'une émulsion solvant immiscible dans l'eau toutefois susceptible de s'inverser selon les proportions des deux phases en présence, entre une solution aqueuse de tannin condensé et une huile végétale ou entre une solution aqueuse de tannin condensé et un solvant volatil immiscible dans l'eau, et ont montré pour la première fois que des tannins condensés peuvent être mis en oeuvre directement dans un procédé d'émulsification.

Dans un mode de réalisation avantageux de l'invention, lorsqu'on souhaite préparer des polyHIPEs, la première phase liquide peut contenir un agent antimousse représentant moins de 1 % en volume. Cet agent peut être n'importe quel agent antimousse connu de l'homme du métier ; il sera avantageusement sous forme liquide. On peut citer à titre d'exemple le polydiméthylsiloxane. En l'absence d'agent antimousse, la vitesse d'agitation doit être contrôlée si l'on souhaite éviter l'aération de la solution. En présence d'agent antimousse, la vitesse d'agitation n'a plus autant d'importance puisqu'il n'y a plus de risque d'aération de la solution. Dans tous les cas, l'adaptation de ces paramètres est à la portée de l'homme du métier. La vitesse sera choisie de telle sorte que l'émulsion ne soit pas déstabilisée et sera fonction du tensioactif utilisé. Avantageusement la vitesse d'agitation, lors du mélange des deux phases, sera comprise entre 200 et 2000 t/min lorsqu'on utilise un mélangeur à hélice.

Dans un autre mode de réalisation avantageux de l'invention, la concentration en tannin condensé dans la solution aqueuse est comprise entre 20 et 60 % en masse de la masse totale (tannin condensé + eau), avantageusement entre 30 et 60 % en masse et est avantageusement égale à 40 % en masse de la masse totale (tannin condensé + eau).

Le pH de la solution de tannin condensé est typiquement de l'ordre de 4,5 ; il peut être ajusté à d'autres valeurs comprises entre 2 et 8 soit par ajout d'un acide, avantageusement l'acide para-toluène sulfonique, soit par ajout d'une base, avantageusement la soude. De manière avantageuse le pH est compris entre 2 et 6.

Dans un autre mode de réalisation avantageux du procédé de l'invention, lorsque la première phase liquide est une phase aqueuse de tannin condensé et la seconde phase une huile végétale ou un solvant volatil non miscible à l'eau, le rapport huile/solution aqueuse de tannin condensé ou solvant volatil non miscible à l'eau /solution aqueuse de tannin condensé est compris entre 0,4/1 et 4/1 en volume.

Dans encore un autre mode de réalisation avantageux du procédé de l'invention, lorsque la première phase liquide est une phase aqueuse de tannin condensé et la seconde phase est un mélange d'huile végétale et d'air ou de solvant volatil non miscible à l'eau et d'air, le rapport huile/solution aqueuse de tannin condensé ou solvant volatil non miscible à l'eau/solution aqueuse de tannin condensé est compris entre 0,3/1 et 4/1 en volume. Le rapport air/émulsion peut prendre toute valeur jusqu'à 2/1 en volume, et avantageusement avoir un rapport 1/1 en volume. Autrement dit, le volume total avant polymérisation est avantageusement le double de celui des liquides (solution aqueuse de tannin condensé et huile végétale ou solvant volatil non miscible à l'eau) initialement incorporés.

Conformément à l'invention, le tannin condensé peut être avantageusement choisi dans le groupe comprenant les tannins de type prodelphinidine, de type procyanidine, de type prorobinetinidine, de type profisetinidine, et toute combinaison desdits quatre types de tannin en toutes proportions. Avantageusement ledit tannin condensé est choisi parmi le tannin de mimosa, le tannin de pin et le tannin de quebracho.

On peut utiliser, conformément à l'invention, tout tensioactif connu de l'homme du métier et choisi dans le groupe comprenant les tensioactifs non ioniques, présentant une balance hydrophile/lipophile supérieure à 7, notamment supérieure à 10. Avantageusement on utilise de l'huile de ricin éthoxylée ou du Polysorbate 80.

Le durcisseur est choisi parmi ceux habituellement utilisés dans ce type de procédé et peut être avantageusement choisi dans le groupe comprenant les aldéhydes, les oxazolidines, les nitroparaffines, l'alcool furfurylique, ainsi que toute combinaison de ces durcisseurs entre eux en toutes proportions. A titre d'exemple d'aldéhydes ou de composés susceptibles de se décomposer en aldéhydes, on peut citer l'hexaméthylènetétramine (HMT), le formaldéhyde, le paraformaldéhyde, le furfural, le glutaraldéhyde et le glyoxal ; à titre d'exemple de nitroparaffines on peut citer notamment le trihydroxyméthylnitrométhane et les composés homologues. La nature et la quantité du durcisseur sont liées à la nature du tannin condensé utilisé et l'homme du métier saura choisir, à la lumière de ses connaissances générales, le type de durcisseur et la quantité. Le durcisseur peut être utilisé sous forme liquide ou sous forme d'une poudre et l'homme du métier saura choisir la forme la plus appropriée. L'hexaméthylènetétramine sera utilisée de préférence en poudre.

La dispersion de la seconde phase dans la première phase et l'obtention de l'émulsion, peut se faire par toute technique connue de l'homme du métier, par exemple par le procédé décrit dans la thèse de Claire Forgacz (http://www.theses.fr/2011BOR14435).

Dans un mode de réalisation avantageux de l'invention, pour la préparation de polyHIPES ou de matériau de type polyHIPEs, la première phase liquide est une phase aqueuse de tannin condensé et la seconde phase une huile végétale ou un mélange d'huile végétale et d'air et le procédé comprend les étapes suivantes :
a. préparer une solution de tannin condensé dans l'eau,
b. ajuster le pH de la solution obtenue à l'étape a à une valeur comprise entre 2 et 8,
c. agiter la solution obtenue à l'étape précédente à une vitesse comprise entre 200 et 2000 t/min jusqu'à obtention d'une solution homogène,
d. ajouter le tensioactif et maintenir sous agitation à une vitesse comprise entre 200 et 2000 t/min jusqu'à obtention d'une solution homogène,
e. incorporer goutte à goutte l'huile végétale à la solution obtenue à l'étape d, en maintenant l'agitation du mélange à une vitesse comprise entre 200 et 2000 t/min jusqu'à obtention d'une émulsion stable et homogène,
f. incorporer le durcisseur à mi-durée de l'étape e et poursuivre l'agitation,
g. réaliser la polymérisation à une température supérieure à la température ambiante, avantageusement comprise entre 40 et 90°C, encore plus avantageusement égale à 85 °C, jusqu'à l'obtention d'une émulsion solide,
h. laver le solide obtenu à l'étape précédente avec un solvant organique,
i. sécher le monolithe polyHIPE ou de type polyHIPE.

La nature de la seconde phase huile végétale seule ou mélange d'huile végétale et d'air est déterminée par la sévérité de l'agitation, par exemple au travers de la vitesse de rotation de l'agitateur à hélice, et surtout, dans le cas des polyHIPEs, par la présence ou non d'antimousse. Ainsi, au-delà de typiquement 500 t/min, en l'absence d'antimousse, de l'air est mécaniquement incorporé en plus de l'huile, et les bulles sont stabilisées par le tensioactif en présence. Dans ce cas, le volume total est supérieur à la somme des deux, et cela d'autant plus que la vitesse est élevée et maintenue longtemps. En revanche à faible vitesse en l'absence d'antimousse, typiquement aux environs de 250 t/min, seule de l'huile est incorporée donc le volume total est égal au volume solution de tannin plus le volume huile.

Conformément à l'invention, le procédé décrit ci-dessus peut être réalisé dans les mêmes conditions en utilisant, comme seconde phase, soit un solvant volatil non miscible à l'eau, soit un mélange de solvant volatil non miscible à l'eau et d'air. Dans ce cas l'étape h) de lavage n'est pas nécessaire, le solvant volatil étant éliminé par l'étape i) de séchage.

Dans un autre mode de réalisation avantageux du procédé dans lequel la première phase liquide est une phase aqueuse de tannin condensé et la seconde phase une huile végétale ou un solvant volatil non miscible dans l'eau, la solution de tannin mise en oeuvre à l'étape a peut comprendre un agent antimousse tel que défini précédemment et le tensioactif être ajouté non pas à la solution de tannin condensé mais à l'huile végétale ou au solvant volatil non miscible dans l'eau.

Le monolithe obtenu est rendu hautement poreux soit par l'extraction de l'huile quand on utilise une seconde phase uniquement huileuse, soit par évaporation quand la seconde phase est un solvant volatil non miscible à l'eau, soit par extraction de l'huile et incorporation d'air quand la seconde phase est un mélange d'huile et d'air, soit par évaporation du solvant et incorporation d'air quand la seconde phase est un mélange solvant volatil non miscible à l'eau et d'air.

Le solvant organique utilisé à l'étape h) de lavage peut être n'importe quel solvant connu de l'homme du métier apte à dissoudre et extraire l'huile. On peut citer à titre d'exemple le méthanol, l'éthanol et l'acétone. On privilégiera l'éthanol, plus compatible avec la notion de matériau vert.

La vitesse d'agitation est un des paramètres qui permet de contrôler la distribution de la taille des pores et sera fixée en fonction de la porosité souhaitée. L'homme du métier saura définir à la lumière de ses connaissances et par des tests de routine la vitesse nécessaire.

Dans une variante du procédé selon l'invention, il est possible de préparer les matériaux monolithiques poreux polyHIPE selon les étapes suivantes :
a. préparer un mélange contenant de l'eau, de l'huile végétale, un durcisseur, un tensioactif et éventuellement un agent antimousse,
b. ajuster le pH du mélange obtenu à l'étape a, à une valeur comprise entre 2 et 8,
c. agiter le mélange obtenu à l'étape précédente à une vitesse comprise entre 200 et 2000 t/min jusqu'à obtention d'une émulsion stable et homogène,
d. ajouter le tannin condensé en poudre à l'émulsion obtenue à l'étape c et agiter entre 200 et 2000 t/min jusqu'à obtention d'une nouvelle émulsion stable et homogène,
e. réaliser la polymérisation à une température supérieure à la température ambiante, avantageusement comprise entre à 40 et 90 °C, encore plus avantageusement égale à 85 °C, jusqu'à l'obtention d'une émulsion solide,
f. laver le solide obtenu à l'étape précédente avec un solvant organique apte à dissoudre et extraire l'huile,
g. sécher le monolithe rendu hautement poreux.

Comme pour le mode de réalisation précédent, c'est la sévérité de l'agitation, par exemple au travers de la vitesse de rotation de l'agitateur à hélice, et la présence ou non d'antimousse, qui définit la nature de la seconde phase.

Dans ce dernier mode de réalisation, on peut remplacer l'huile utilisée à l'étape a par un solvant volatil non miscible à l'eau. Dans ce cas, il ne sera pas nécessaire de réaliser l'étape f de lavage, le solvant volatil étant éliminé par l'étape g de séchage.

Dans une variante du procédé selon l'invention, il est possible de préparer les matériaux monolithiques poreux de type polyHIPE selon les étapes suivantes :
a. préparer un mélange contenant de l'eau, de l'huile végétale, un durcisseur, et un tensioactif,
b. ajuster le pH du mélange obtenu à l'étape a, à une valeur comprise entre 2 et 8,
c. agiter le mélange obtenu à l'étape précédente à une vitesse comprise entre 200 et 2000 t/min jusqu'à obtention d'une émulsion stable et homogène,
d. ajouter le tannin condensé en poudre à l'émulsion obtenue à l'étape c et agiter entre 200 et 2000 t/min jusqu'à obtention d'une nouvelle émulsion stable et homogène,
e. réaliser la polymérisation à une température supérieure à la température ambiante, avantageusement comprise entre à 40 et 90 °C, encore plus avantageusement égale à 85 °C, jusqu'à l'obtention d'une émulsion solide,
f. laver le solide obtenu à l'étape précédente avec un solvant organique apte à dissoudre et extraire l'huile,
g. sécher le monolithe rendu hautement poreux.

Dans ce mode de réalisation c'est la sévérité de l'agitation, par exemple au travers de la vitesse de rotation de l'agitateur à hélice, qui définit la nature de la seconde phase.

Dans toutes les variantes du procédé, suite à l'agitation mécanique, sont incorporés dans l'émulsion solide soit de l'huile seule, soit du solvant non miscible à l'eau seul, soit un mélange d'huile et d'air, soit un mélange de solvant non miscible à l'eau et d'air suivant la nature de la seconde phase qui a été utilisée.

L'agitation mécanique dans les différentes étapes peut être réalisée par tout agitateur ou toute méthode à la disposition de l'homme du métier, notamment les agitateurs à pales ou par la méthode des pistons en opposition.

Le monolithe obtenu est rendu hautement poreux soit par l'extraction de l'huile quand on utilise une seconde phase uniquement huileuse, soit par extraction de l'huile et incorporation d'air quand la seconde phase est un mélange d'huile et d'air, soit par évaporation du solvant lors du séchage lorsqu'on utilise un solvant volatil non miscible à l'eau, soit par évaporation du solvant lors du séchage et incorporation d'air quand la seconde phase est un mélange d'un solvant volatil non miscible à l'eau et d'air.

Le solvant utilisé à l'étape f de lavage peut être n'importe quel solvant connu de l'homme du métier apte à dissoudre et extraire l'huile. On peut citer à titre d'exemple le méthanol, l'éthanol et l'acétone. On privilégiera l'éthanol, plus compatible avec la notion de matériau vert.

Dans un autre mode de réalisation du procédé de l'invention, pour la préparation de mousses rigides, la première phase liquide est une phase aqueuse de tannin condensé et la seconde phase de l'air et le procédé comprend les étapes suivantes :
a. préparer une solution de tannin condensé dans l'eau,
b. ajuster le pH de la solution obtenue à l'étape a, à une valeur comprise entre 2 et 8,
c. agiter la solution obtenue à l'étape précédente à une vitesse comprise entre 400 et 600 t/min jusqu'à obtention d'une solution homogène,
d. ajouter le tensioactif à la solution obtenue à l'étape c et maintenir sous agitation à une vitesse comprise entre 200 et 2000 t/min jusqu'à l'obtention d'une mousse liquide homogène,
e. incorporer le durcisseur à mi-durée de l'étape d et poursuivre l'agitation,
f. réaliser la polymérisation à une température supérieure à la température ambiante, avantageusement comprise entre 40 et 90 °C, encore plus avantageusement égale à 85 °C pendant au moins 24 heures,
g. sécher le monolithe.

Dans les différents modes de réalisation décrits précédemment, l'étape d de préparation de l'émulsion peut durer typiquement pendant plusieurs minutes à plusieurs dizaines de minutes, avantageusement pendant 45 min.

Les matériaux obtenus par le procédé de l'invention sont nouveaux et font également partie de l'invention.

Aussi l'invention a également pour objet, un matériau monolithique poreux polyHIPE, de type polyHIPE ou sous forme de mousse rigide, susceptible d'être obtenu par polymérisation de tannins condensés par un procédé tel que décrit précédemment.

L'émulsion à base de tannins condensés et la mousse liquide homogène sont nouvelles et font également partie de l'invention.

Aussi l'invention a pour objet une émulsion, notamment utile pour la préparation d'un matériau monolithe poreux, comprenant une première phase qui est une solution aqueuse de tannins condensés contenant un durcisseur, et une seconde phase, à base d'huile notamment d'huile végétale ou d'un solvant volatil non miscible à l'eau, ou d'un mélange d'huile et d'air, notamment d'huile végétale et d'air, ou d'un mélange de solvant volatil non miscible à l'eau et d'air, et l'une au moins desdites phases comprenant un tensioactif.

L'invention a également pour objet une mousse liquide, notamment utile pour la préparation d'un matériau monolithe poreux, comprenant une première phase qui est une solution aqueuse de tannins condensés contenant un tensioactif et un durcisseur et une seconde phase qui est de l'air.

Les matériaux monolithiques poreux polyHIPE et de type polyHIPE présentent une structure poreuse ouverte et interconnectée dont la densité apparente est comprise entre 0,03 et 0,5 g/cm³.

Les mousses rigides présentent une densité apparente pouvant aller jusqu'à 0,25 g/cm³.

Ces matériaux sont isolants thermiquement. Ils ne brûlent pas mais se consument lentement si une grande quantité de chaleur est apportée. Ils sont auto-extinguibles.

Ils peuvent être soumis à une pyrolyse de manière à donner des mousses, des polyHIPEs et des matériaux de type polyHIPE en carbone vitreux.

Aussi un autre objet de l'invention est un monolithe carboné poreux susceptible d'être obtenu par pyrolyse d'un matériau monolithique poreux selon l'invention.

Les monolithes de l'invention présentent l'intérêt d'avoir une composition chimique simple puisqu'ils sont constitués uniquement de matière organique ; ils sont donc également fonctionnalisables afin d'être utilisables comme support de catalyseur.

Aussi un autre objet de l'invention est un monolithe organique poreux fonctionnel, notamment doté de propriétés catalytiques, susceptible d'être obtenu par fonctionnalisation d'un matériau monolithique poreux selon l'invention.

Ils peuvent être mis en contact, soit avant pyrolyse avec des dérivés organiques du silicium, soit après pyrolyse avec des dérivés organiques du silicium ou du silicium liquide et/ou gazeux afin d'être convertis en mousse de carbure de silicium de même structure.

Aussi la présente invention a pour objet un monolithe poreux en carbure de silicium (SiC) ou composite Si - SiC susceptible d'être obtenu :
a. soit par imprégnation par des polymères précéramiques puis pyrolyse d'un matériau monolithique poreux selon l'invention,
b. soit par infiltration de silicium liquide ou gazeux d'un monolithe carboné poreux selon l'invention,
c. soir par combinaison des deux méthodes.

Les matériaux monolithiques poreux selon l'invention peuvent être utilisés dans le domaine de la catalyse, de la chromatographie, de l'isolation thermique ou phonique, de l'amortissement des chocs, de l'ingénierie tissulaire et de la libération de médicaments. Les mousses solides peuvent également être utilisées comme mousses florales.

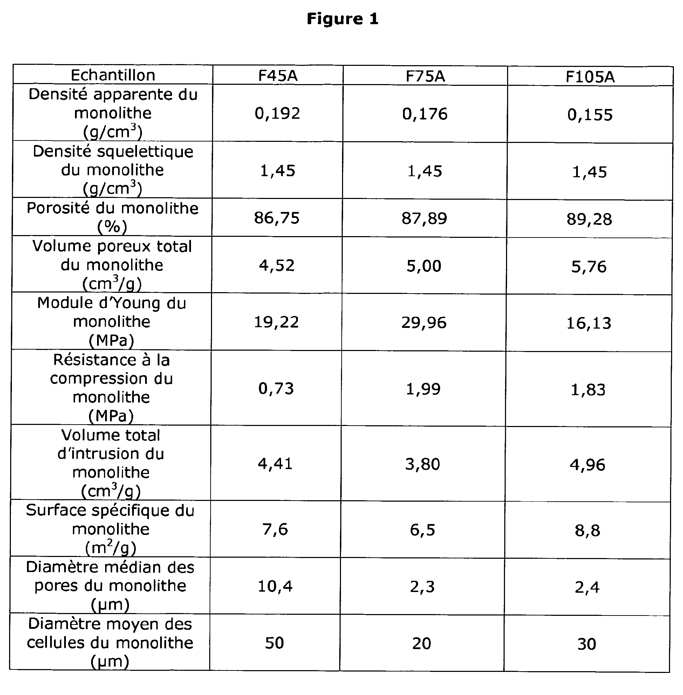

L'invention est illustrée par les exemples 1 à 9 et les figures 1 à 22 qui suivent.

Dans les figures qui suivent, le volume total d'intrusion des monolithes et la surface spécifique des monolithes sont mesurés par intrusion de mercure jusqu'à une pression de 4 MPa. Le diamètre médian des pores des monolithes est déterminé par intrusion de mercure comme le diamètre de pore auquel 50% du volume poreux total est rempli par le mercure et le diamètre moyen des cellules des monolithes est déterminé à partir d'images de microscopie électronique.
La figure 1 résume les propriétés de monolithes polyHIPE préparés à partir d'une solution de tannin de mimosa à pH 2,5 et des concentrations en huile de tournesol variables selon l'exemple 1. F45A : 45 ml d'huile de tournesol /40 ml de solution de tannin de mimosa à 40 % en masse par rapport à la masse totale (eau + tannin de mimosa) ; F75A : 75 ml d'huile de tournesol /40 ml de solution de tannin de mimosa à 40 % en masse par rapport à la masse totale (eau + tannin de mimosa) ; F105A : 105 ml d'huile de tournesol/40 ml de solution de tannin de mimosa à 40 % en masse par rapport à la masse totale (eau + tannin de mimosa).
La figure 2 présente des clichés de microscopie électronique à balayage, à 2 grossissements différents, des monolithes décrits en figure 1.

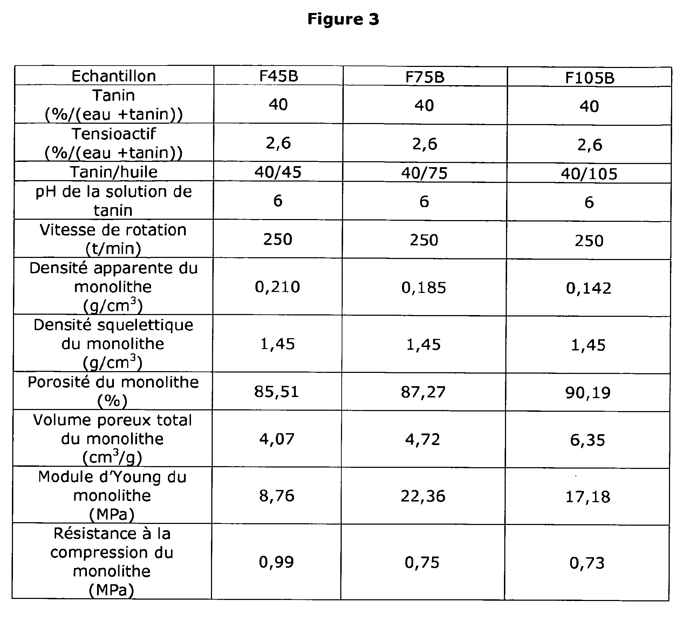

La figure 3 résume les propriétés de monolithes polyHIPE préparés à partir d'une solution de tannin de mimosa à pH 6 et des concentrations en huile de tournesol variables selon l'exemple 1. F45B : 45 ml d'huile de tournesol /40 ml de solution de tannin de mimosa à 40 % en masse par rapport à la masse totale (eau + tannin de mimosa) ; F75B : 75 ml d'huile de tournesol /40 ml de solution de tannin de mimosa à 40 % en masse par rapport à la masse totale (eau + tannin de mimosa) ; F105B : 105 ml d'huile de tournesol/40 ml de solution de tannin de mimosa à 40 % en masse par rapport à la masse totale (eau + tannin de mimosa).

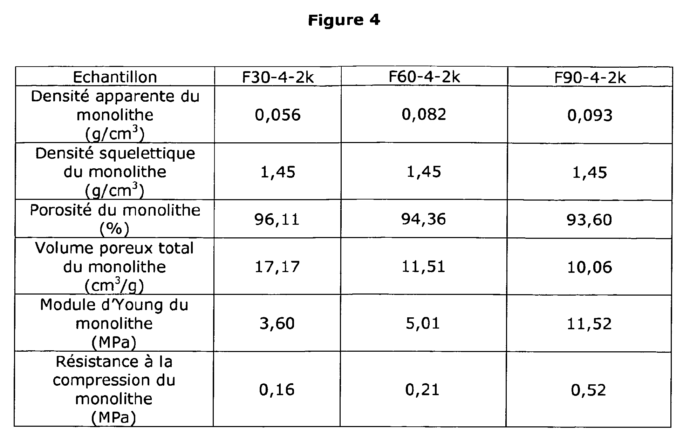

La figure 4 résume les propriétés de monolithes aérés de type polyHIPE préparés à partir d'une solution de tannin de mimosa à pH 4 et des concentrations en huile de tournesol variables selon l'exemple 2. F30-4-2k : 30 ml d'huile de tournesol/40 ml de solution de tannin de mimosa à 40 % en masse par rapport à la masse totale (eau + tannin de mimosa) ; F60-4-2k : 60 ml d'huile de tournesol/40 ml de solution de tannin de mimosa à 40 % en masse par rapport à la masse totale (eau + tannin de mimosa) ; F90-4-2k : 90 ml d'huile de tournesol/40 ml de solution de tannin de mimosa à 40 % en masse par rapport à la masse totale (eau + tannin de mimosa).
La figure 5 présente des clichés de microscopie électronique à balayage, à 2 grossissements différents, des monolithes décrits en figure 4.

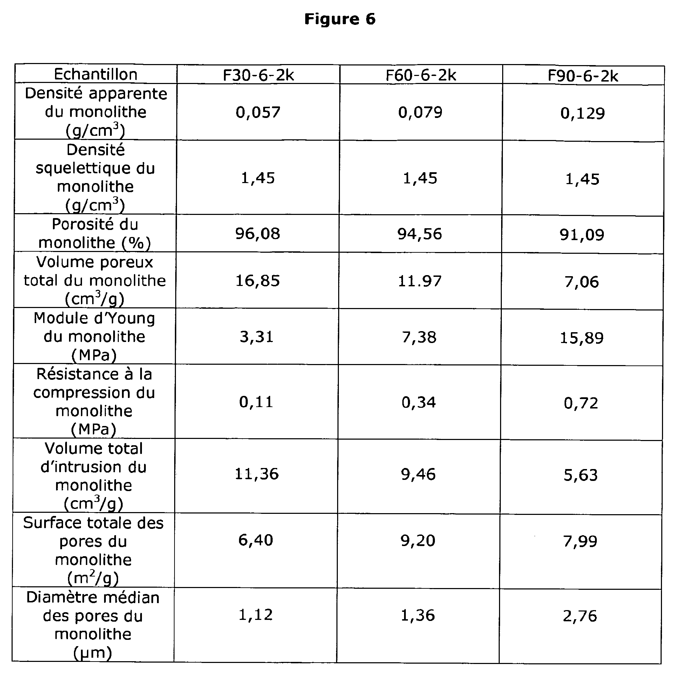

La figure 6 résume les propriétés de monolithes aérés de type polyHIPE préparés à partir d'une solution de tannin de mimosa à pH 6 et des concentrations en huile de tournesol variables selon l'exemple 2. F30-6-2k : 30 ml d'huile de tournesol/40 ml de solution de tannin de mimosa à 40 % en masse par rapport à la masse totale (eau + tannin de mimosa) ; F60-6-2k : 60 ml d'huile de tournesol/40 ml de solution de tannin de mimosa à 40 % en masse par rapport à la masse totale (eau + tannin de mimosa) ; F90-6-2k : 90 ml d'huile de tournesol/40 ml de solution de tannin de mimosa à 40 % en masse par rapport à la masse totale (eau + tannin de mimosa).

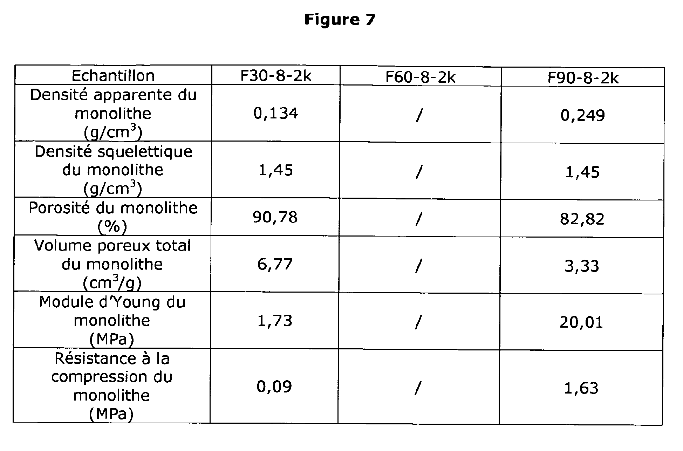

La figure 7 résume les propriétés de monolithes aérés de type polyHIPE préparés à partir d'une solution de tannin de mimosa à pH 8 et des concentrations en huile de tournesol variables selon l'exemple 2. F30-8-2k : 30 ml d'huile de tournesol/40 ml de solution de tannin de mimosa à 40 % en masse par rapport à la masse totale (eau + tannin de mimosa) ; F60-8-2k : 60 ml d'huile de tournesol/40 ml de solution de tannin de mimosa à 40 % en masse par rapport à la masse totale (eau + tannin de mimosa) ; F90-8-2k : 90 ml d'huile de tournesol/40 ml de solution de tannin de mimosa à 40 % en masse par rapport à la masse totale (eau + tannin de mimosa).
La figure 8 résume les propriétés de monolithes de type mousse préparés à partir d'une solution de tannin de mimosa à pH 2,8 et à des concentrations variables selon l'exemple 3. F20-40 : 30 ml de solution de tannin de mimosa à 20 % en masse par rapport à la masse totale (eau + tannin de mimosa) ; F40-40 : 30 ml de solution de tannin de mimosa à 40 % en masse par rapport à la masse totale (eau + tannin de mimosa) ; F50-40 : 30 ml de solution de tannin de mimosa à 50 % en masse par rapport à la masse totale (eau + tannin de mimosa).
La figure 9 présente des clichés de microscopie électronique à balayage des monolithes décrits en figure 8.
La figure 10 résume les propriétés des monolithes carbonés poreux CF45A, CF75A et CF105A préparés selon l'exemple 4, respectivement à partir de F45A, F75A et F105A de l'exemple 1.
La figure 11A montre les monolithes poreux avant carbonisation (à gauche F60-4-2k et à droite F30-4-2k) et la figure 11B, les monolithes carbonés poreux correspondants (à gauche CF60-4-2k et à droite CF30-4-2k), obtenus selon l'exemple 4 à partir de F60-4-2k et de F30-4-2k de l'exemple 2.
La figure 12 résume les propriétés des monolithes carbonés poreux CF30-6-2k, CF60-6-2k et CF90-6-2k préparés selon l'exemple 4, respectivement à partir de F30-6-2k, F60-6-2k et F90-6-2k de l'exemple 2.

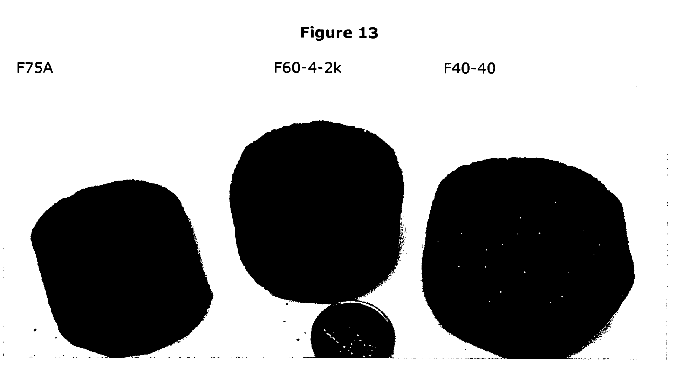

La figure 13 présente les trois types de matériaux organiques dérivés de tannin de mimosa (donc non carbonisés) obtenus selon les procédés de l'invention : à gauche monolithe poreux polyHIPE, F75A, obtenu conformément à l'exemple 1, à partir d'une émulsion d'une solution aqueuse de tannin de mimosa avec de l'huile de tournesol (porosité environ 88 %), au centre monolithe poreux de type polyHIPE, F60-4-2k, obtenu conformément à l'exemple 2, à partir d'une émulsion à base d'une solution aqueuse de tannin de mimosa et d'huile de tournesol, dans laquelle de l'air a été incorporé mécaniquement (porosité environ 94 %), et à droite monolithe poreux de type mousse, F40-40, obtenu conformément à l'exemple 3 à partir d'une mousse liquide formée à partir d'une solution aqueuse de tannin de mimosa et d'air (porosité environ 97 %).
La figure 14 résume les propriétés des monolithes cellulaires polyHIPEs préparés selon l'exemple 5. TC monolithe préparé avec du cyclohexane comme seconde phase liquide ; TH monolithe préparé avec de l'huile de tournesol comme seconde phase liquide.
La figure 15 présente les trois types de monolithes cellulaires polyHIPEs préparés selon l'exemple 5. Cyclohexane : monolithe préparé avec du cyclohexane comme seconde phase liquide ; Heptane : monolithe préparé avec de l'heptane comme seconde phase liquide ; Huile de tournesol : monolithe préparé avec de l'huile de tournesol comme seconde phase liquide.
La figure 16 résume les propriétés des monolithes de type mousse préparés selon l'exemple 6 en utilisant du Tween 80 comme tensioactif.
La figure 17 présente deux types de monolithes de type mousse préparés selon l'exemple 6. Pluronic 6800 : mousse préparée avec du Pluronic 6800 comme tensioactif et Tween 80 : mousse préparée avec du Tween 80 comme tensioactif.
La figure 18 présente cinq types de monolithes cellulaires polyHIPEs préparés selon l'exemple 7 obtenus à partir de formulations contenant différentes concentrations de durcisseur.
La figure 19 résume les propriétés des monolithes cellulaires polyHIPEs préparés selon l'exemple 8 en utilisant du Triton X 100 comme tensioactif.
La figure 20 présente trois types de monolithes cellulaires polyHIPEs préparés selon l'exemple 8 obtenus à partir de formulations contenant différents types de tensioactifs : Tween 80, Triton X100 et Cremophor ELP.
La figure 21 résume les propriétés des monolithes cellulaires polyHIPEs préparés selon l'exemple 9 ; TFA2 tensioactif Cremophor ELP à 2, % par rapport à la masse totale de la solution et TFA2G : influence d'éthylène glycol à une concentration de 5 % par rapport à la masse totale de la solution.
La figure 22 présente cinq types de monolithes cellulaires polyHIPEs préparés selon l'exemple 9 obtenus à partir de formulations contenant de l'alcool furfurylique comme durcisseur et différentes quantité de tensioactif (2, 4, 6, 8 et 10 % de Cremophor ELP par rapport à la masse totale de la solution).

### Exemple 1: Monolithes poreux polyHIPE préparés à partir d'émulsion : huile de tournesol/eau, sans air

### 1.1. Mode opératoire

### Réactifs utilisés :

- Tannin de Mimosa commercial utilisé tel quel
- Eau
- Durcisseur : solution aqueuse à 30 % en poids d'hexaméthylènetétramine (hexamine)
- Tensioactif : Cremophor® ELP (huile de ricin éthoxylée)
- Huile de tournesol
- Soude 2M ou acide para-toluènesulfonique (PTSA) en poudre

Des monolithes sont préparés à partir des émulsions suivantes :

| Emulsion | | F45A | F75A solution de référence | F105A |
|---|---|---|---|---|
| Solution de tannin | Tannin (g) | 20 | 20 | 20 |
| | Eau (g) | 30 | 30 | 30 |
| | Tannin/(eau +tannin) (%) | 40 | 40 | 40 |
| | Tensioactif (g) | 1,33 | 1,33 | 1,33 |
| | Tensioactif/ (eau +tannin) (%) | 2,6 | 2,6 | 2,6 |
| pH de la solution de tannin | | 2,5 | 2,5 | 2,5 |
| Tannin/huile (mL/mL) | | 40/45 | 40/75 | 40/105 |
| Vitesse de rotation (t/min) | | 250 | 250 | 250 |

Les différentes étapes sont les suivantes :

### 1^{ère} étape - préparation de la solution de tannin de mimosa

Une solution de tannin de mimosa est préparée en ajoutant le tannin de mimosa dans l'eau. Le pH de la solution est ajusté avec de la soude 2M ou de l'acide paratoluène sulfonique (PTSA). Le mélange est agité mécaniquement avec un agitateur à hélice muni d'une hélice à 3 pales à 250 t/min pendant 10 minutes pour obtenir une solution très homogène.

### 2^{ème} étape - addition du tensioactif

Le tensioactif est ajouté à la solution de tannin de mimosa obtenue à l'étape précédente et le mélange agité à 250 t/min pendant 20 minutes ; une solution brune homogène est obtenue.

### 3^{ème} étape - addition de l'huile de tournesol

L'huile de tournesol est ajoutée goutte à goutte, sous agitation à 250 t/min, à la vitesse de 44 gouttes/min. Pendant que l'huile est ajoutée, 4,47 g de la solution d'hexamine est également ajoutée au mélange. Pendant l'addition de l'hexamine, la vitesse d'agitation est momentanément augmentée pendant environ 30 s à 900 t/min afin de faciliter la dissolution de l'hexamine puis ramenée à 250 t/min.

### 4^{ème} étape - chauffage du mélange

Le récipient contenant le mélange obtenu à l'étape précédente est couvert par un film plastique ou par un film d'aluminium, afin d'éviter que l'émulsion ne sèche en surface, et placé dans une étuve ventilée à 85 °C pendant 20 heures. La gélification est très rapide, environ 15 minutes pour toutes les formulations. Cependant 20 heures à 85°C sont nécessaires pour avoir des réactions complètes de réticulation et obtenir des monolithes totalement durcis.

### 5^{ème} étape - lavage des échantillons dans l'acétone

Après 20 heures de chauffage, les monolithes durcis sont sortis de l'étuve et laissés refroidir à la température ambiante. Les monolithes sont ensuite découpés sous forme de cylindres, placés dans un extracteur Soxhlet puis lavés par de l'acétone chaude à reflux pendant 7 jours.

### 6^{ème} étape - séchage des échantillons

Après 7 jours de lavage, les échantillons sont séchés à la température ambiante pendant 7 jours.

### 7 ^{ème} étape - mesure des caractéristiques physiques des échantillons

Ces caractéristiques sont mesurées par des techniques connues de l'homme du métier.

### 1.2. Résultats

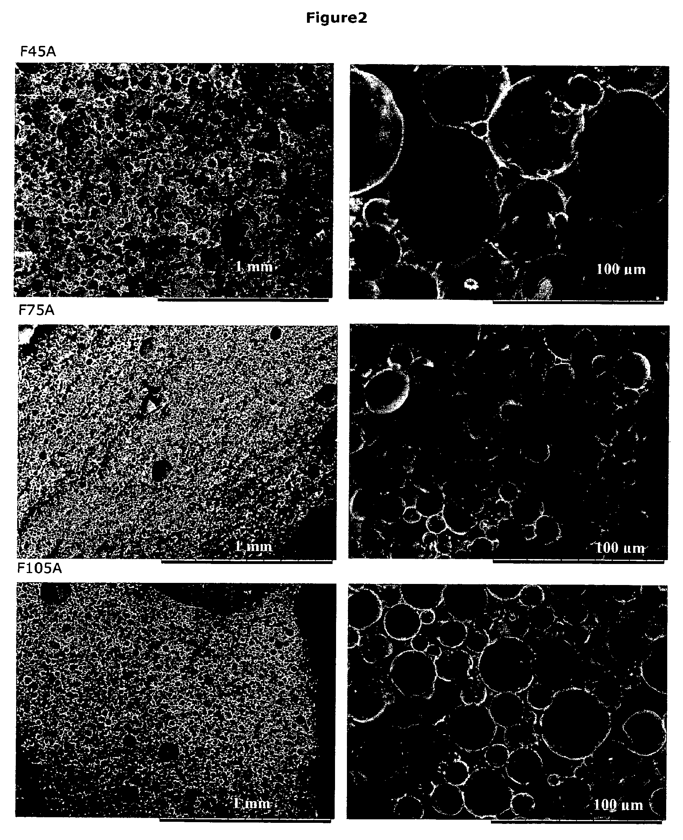

Ils sont donnés dans les tableaux des figures 1 et 3 et à la figure 2 pour des monolithes préparés à partir d'une solution de tannin de mimosa à 20 g de tannin de mimosa dans 30 g d'eau. Sur la base des figures 1 à 3, on peut observer que :
- la porosité des monolithes est très élevée, de l'ordre de 90%, ce qui est remarquable pour ce type de matériaux,
- la porosité augmente avec la fraction d'huile de tournesol incorporée dans l'émulsion, sans toutefois qu'il y ait proportionnalité. C'est une caractéristique fréquente des polyHIPE qui s'explique d'une part par le retrait des matériaux pendant les phases de durcissement et de séchage, et d'autre part par le changement du type d'émulsion : de « huile dans eau » à « eau dans huile », quand la fraction volumique d'huile augmente,
- l'inversion de l'émulsion est observée lorsque l'on compare les images des matériaux F45A et F105A. F45A présente la structure cellulaire typique d'un polyHIPE, i.e. montre une phase solide continue présentant des cellules plutôt sphériques, connectées entre elles par des fenêtres plutôt circulaires. Les brins, de section triangulaire, sont pleins. La structure poreuse est donc hiérarchisée : cellulaire à l'échelle macroscopique, avec des interconnexions beaucoup plus étroites et très nombreuses dans les parois des cellules. F105 a également une structure cellulaire, mais basée sur un empilement de sphères creuses ouvertes par des fenêtres. Les brins, de section triangulaire, sont creux. F75A montre également ce type de structure,
- les diamètres moyens des cellules et des connexions (les « pores ») évoluent de manière complexe avec la porosité totale, en lien avec les changements de structure observés en microscopie électronique. Ces changements s'expliquent par : (i) l'inversion de l'émulsion au-delà d'une quantité critique d'huile de tournesol ; (ii) la compétition entre porosité créée par l'huile de tournesol et perdue par le retrait au cours du procédé ; (iii) le fait que le taux de tensioactif soit resté constant dans ces exemples, en dépit de fractions d'huile de tournesol très différentes dans l'émulsion,
- en conséquence, les propriétés mécaniques ne présentent pas, pour les seuls exemples donnés ici, d'évolution régulière. Elles sont remarquablement élevées, compte tenu des valeurs correspondantes de porosité totale.

### Exemple 2 : Monolithes poreux aérés de type polyHIPE préparés à partir d'émulsion : huile de tournesol et air / eau

### 2.1 Mode opératoire

Des monolithes sont préparés à partir des émulsions suivantes :

| Emulsion | | F30-4-2k | F30-6-2k | F30-8-2k | F60-4-2k | F60-6-2k | F60-8-2k | F90-4-2k | F90-6-2k | F90-8-2k |
|---|---|---|---|---|---|---|---|---|---|---|
| Solution de tannin | Tannin (g) | 30 | | | 30 | | | 30 | | |
| | Eau (g) | 20 | | | 20 | | | 20 | | |
| | Tannin/ (eau +tannin) (%) | 15 | | | 15 | | | 15 | | |
| | Tensioactif (g) | 2,33 | | | 2,33 | | | 2,33 | | |
| | Tensioactif/ (eau +tannin) (%) | 4,6 | | | 4,6 | | | 4,6 | | |
| pH de la solution de tannin | | 4 | 6 | 8 | 4 | 6 | 8 | 4 | 6 | 8 |
| Tannin/huile (mL/mL) | | 40/30 | | | 40/60 | | | 40/90 | | |
| Vitesse de rotation (t/min) | | 2000 | | | 2000 | | | 2000 | | |

Les différentes étapes sont les suivantes :

20 g d'eau, 30, 40 ou 60 mL d'huile de tournesol, 4,47 g de la solution aqueuse d'hexamine, 1,3 g de tensioactif et de la soude (quantité nécessaire pour avoir un pH de 4, 6 ou 8) sont mélangés à 2000 t/min jusqu'à obtenir une émulsion blanche, stable et homogène. 30 g de tannin de mimosa sont ajoutés et le mélange est agité pendant 45 minutes à 2000 t/min.

Le durcissement, le lavage et le séchage sont réalisés dans les mêmes conditions que pour les étapes 4 à 6 de l'exemple 1.

### 2.2 Résultats

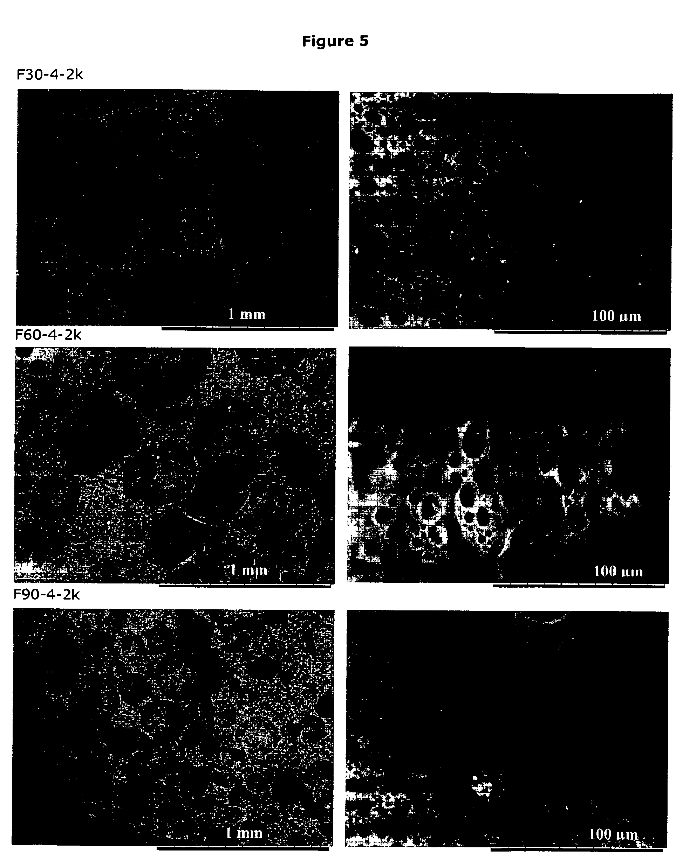

Ils sont donnés dans les tableaux des figures 4, 6 et 7, et à la figure 5. Du fait de l'air incorporé lors de la préparation, un dôme apparait à la surface des échantillons, dôme qui n'apparaît pas dans les monolithes préparés selon l'exemple 1. Sur la base des figures 4 à 7, on peut observer que :
- comme on pouvait s'y attendre, la porosité est plus élevée (i.e. la densité apparente est bien plus faible) que celle des polyHIPEs correspondants préparés avec des proportions tannin de mimosa /huile de tournesol comparables. Ceci s'explique par la présence d'air incorporé dans la structure lors de l'agitation mécanique,
- cet air permet d'obtenir des matériaux monolithiques poreux de structure différente de celle des précédents polyHIPEs à proportions huile de tournesol/solution aqueuse de tannin de mimosa constante. En revanche, des structures qualitativement similaires (cellulaires) aux précédents polyHIPEs peuvent être observées pour des compositions initiales différentes. Cependant, les cellules ont alors des diamètres moyens beaucoup plus grands en présence d'air (comparer la série F45A - F105A avec la série F30-4-2K - F90-4-2K en utilisant le même grossissement : celui pour lequel la barre d'échelle est de 1 mm),
- en conséquence, l'incorporation d'air au travers de l'agitation plus ou moins sévère des phases en présence permet de faire varier considérablement le diamètre moyen des cellules, ce qui n'est pas permis par la variation d'un seul à la fois des paramètres de la formulation,

- la porosité diminue lorsque la proportion d'huile dans l'émulsion augmente. Ce résultat *a priori* inattendu mais bien vérifié dans tous les cas montre que l'air est bien plus facile à incorporer dans une émulsion moins riche en huile de tournesol, et qu'il contribue alors plus aisément à la création de la porosité que l'huile de tournesol ne le fait,
- les propriétés mécaniques restent élevées au regard de la porosité présente, et augmentent systématiquement dès que la porosité diminue. Ce résultat est logique dans la mesure où la structure poreuse ne change pas lorsque la porosité varie,
- en conséquence, les pores sont systématiquement plus larges lorsque la porosité augmente. Cette tendance se voit plus clairement à partir des résultats de porosimétrie au mercure que sur les images de microscopie.

### Exemple 3 : Monolithes poreux préparés à partir de mousses

### 3.1. Mode opératoire

Des monolithes sont préparés comme suit. Une solution aqueuse de tannin de mimosa dont la concentration est de 20, 40 ou 50% de la masse totale (tannin de mimosa + eau) est préparée en mélangeant 7,5 ; 20 ou 30 g de tannin de mimosa, respectivement, avec 30 g d'eau. Cette solution est ensuite mélangée à du PTSA, en quantité suffisante pour atteindre pH 2,8, pendant 10 minutes à 500 t/min. A la fin de cette période, le tensioactif est ajouté et tous les ingrédients sont mélangés à 2000 t/min jusqu'à obtention d'une mousse homogène sans qu'il ne reste de phase liquide condensée, soit 40 min. A la moitié du temps 4,7 g de solution d'hexamine à 30 % sont ajoutés. La mousse obtenue est couverte avec un film et placée dans une étuve ventilée à 85°C pendant 24 h. Ensuite les échantillons sont découpés et mis à sécher à l'air dans une pièce à température ambiante pendant plusieurs jours ou dans la même étuve ventilée pendant quelques heures. Le tableau ci-dessous récapitule les ingrédients et les conditions de préparation.

| Mousse liquide | | F20-40 | F40-40 | F50-40 |
|---|---|---|---|---|
| Solution de tannin | Tannin (g) | 7,5 | 20 | 30 |
| | Eau (g) | 30 | 30 | 30 |
| | tannin/(eau +tannin) (%) | 20 | 40 | 50 |
| | PTSA (g) | 1,12 | 1,12 | 1,12 |
| | Tensioactif (g) | 2,25 | 3 | 3,6 |
| | Tensioactif/ (eau +tannin) (%) | 6 | 6 | 6 |
| pH de la solution de tannin | | 2,8 | 2,8 | 2,8 |
| Temps d'agitation (min) | | 40 | 40 | 40 |
| Vitesse de rotation (t/min) | | 2000 | 2000 | 2000 |

### 3.2. Résultats

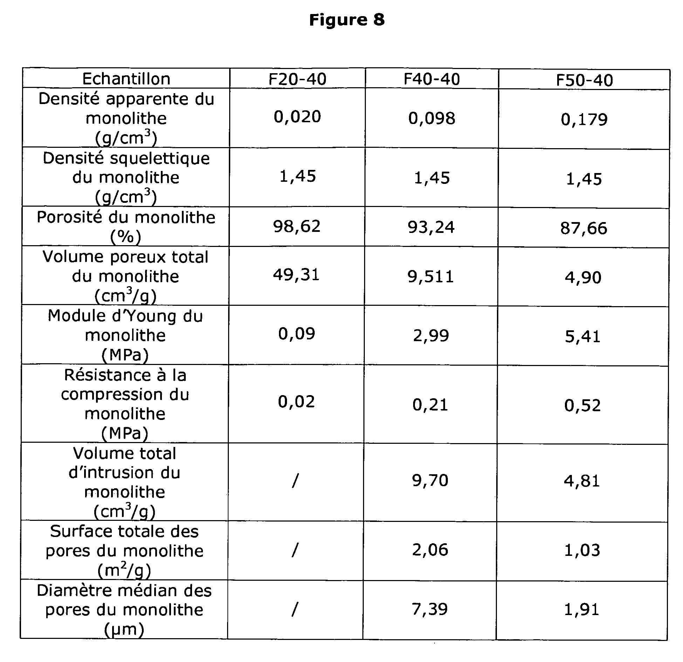

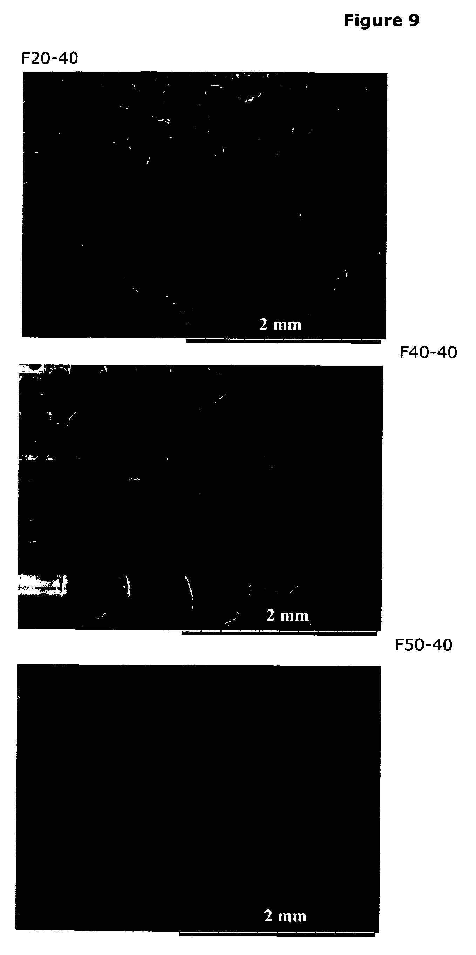

Ils sont donnés dans le tableau de la figure 8, et à la figure 9. Sur la base des figures 8 et 9, on peut observer que :
- la structure poreuse est assez différente de celle des matériaux des exemples 1 et 2 précédents. La structure est en effet celle d'une mousse réticulée, les matériaux obtenus selon les variantes du procédé selon les exemples 1 ou 2 étant davantage cellulaires,
- ainsi, les connexions entre cellules ne sont pas aussi multiples que précédemment car correspondent essentiellement aux parois des cellules qui existaient dans la mousse liquide. La mousse solide n'a conservé de cette structure que les intersections des parois de bulles, autrement dit les brins. Cela est encore confirmé par la surface spécifique, beaucoup plus faible pour les mousses que pour les autres monolithes à porosité équivalente,

- les différences de densité se manifestent dans ces mousses par des changements importants tant de l'épaisseur de la phase solide (les brins) que de diamètre des espaces vides,
- il est beaucoup plus aisé par cette méthode (sans huile) d'obtenir de la porosité très large (la barre d'échelle est ici de 2 mm contre 1 mm dans les précédents clichés) et donc une plus grande porosité,
- la porosité est facilement modifiable par changement de la concentration de la solution de tannin, comme illustré, ou par changement de la nature du tensioactif,
- à porosité totale comparable, les propriétés mécaniques des mousses sont inférieures à celles des monolithes polyHIPE ou de type polyHIPE. Cela s'explique, comme suggéré ci-dessus, par la structure poreuse différente, qui est beaucoup plus ouverte dans le cas des mousses.

### Exemple 4 : Préparation de monolithes carbonés poreux

### 4.1. Mode opératoire

On utilise les monolithes obtenus avec les émulsions F45A, F75A, F105A de l'exemple 1 et les monolithes obtenus avec les émulsions F30-6-2k, F60-6-2k, F90-6-2k de l'exemple 2.

La carbonisation des échantillons est réalisée dans un four tubulaire horizontal à 900 °C pendant 2 h sous atmosphère d'azote (5 °C/min - 50 mL/min).

### 4.2. Résultats

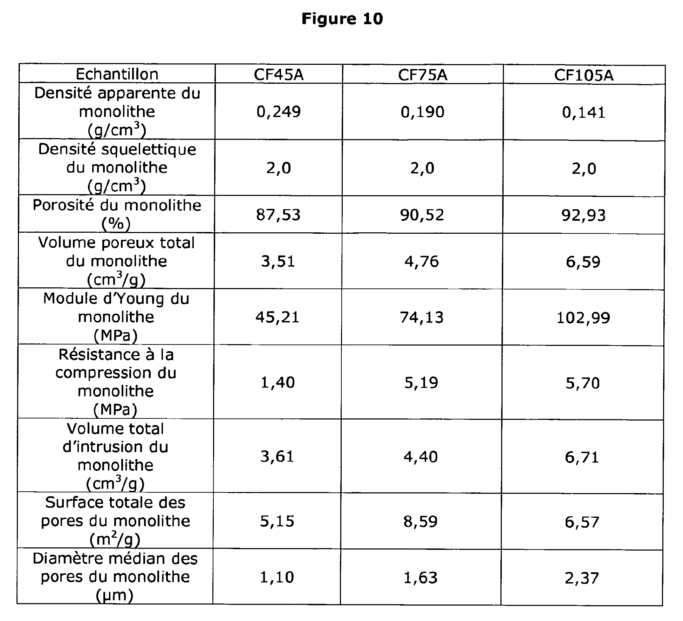

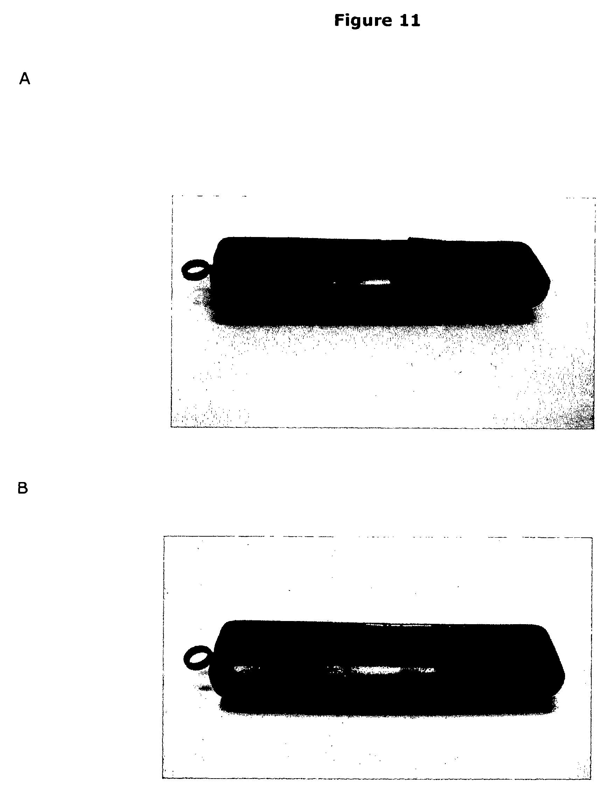

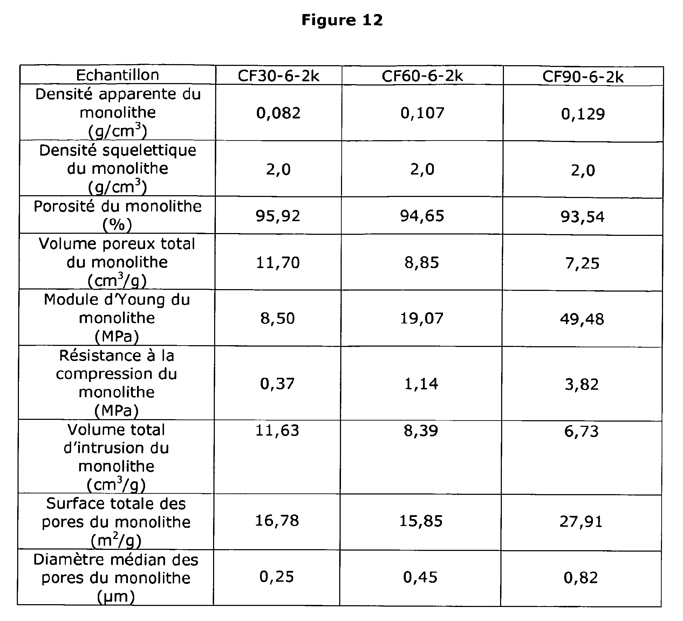

Ils sont donnés dans les tableaux des figures 10 et 12 et à la figure 11. Sur la base des figures 10 à 12, on peut observer que :
- les échantillons résistent très bien au traitement thermique, et les carbones poreux obtenus ont une structure identique à celle des monolithes d'origine. Ils sont exempts de fissures,
- l'évolution de la porosité suit exactement les mêmes tendances que celles des matériaux organiques précurseurs,
- aux incertitudes près (en particulier aux très hautes porosités, > 95%), la porosité des matériaux carbonés est supérieure à celle des précurseurs organiques,
- la pyrolyse ayant engendré une perte de masse et un retrait de grandeur comparable (environ 60%), la densité apparente des matériaux carbonés évolue peu par rapport à celle des précurseurs organiques, aux variations individuelles d'échantillons près (un grand nombre d'échantillons est nécessaire pour l'observer, en raison de la dispersion expérimentale des données),
- en raison du retrait, les pores et les cellules sont toujours plus petits dans les matériaux carbonés,
- le départ de gaz qui accompagne la pyrolyse produit des pores très fins qui font que la surface spécifique des matériaux carbonés est toujours supérieure à celle de leurs homologues organiques
- les propriétés mécaniques sont considérablement augmentées après pyrolyse.

### Exemple 5: Procédé de préparation de monolithes cellulaires polyHIPEs utilisant comme seconde phase liquide un solvant volatil non miscible à l'eau et un durcisseur en poudre plutôt qu'en solution

### 5.1. Mode opératoire

a. 20 g de tannin de mimosa, 1,9 g d'hexaméthylènetétramine (HMT) en poudre et 6 gouttes d'antimousse (polydiméthylsiloxane) sont dissous dans 33,9 g d'eau distillée, puis 0,7 g d'acide paratoluène sulfonique (pTSA) solide sont ajoutés pour ajuster le pH de la solution à 2,5. La fraction massique des solides par rapport à l'eau est donc de 40%. Le mélange est agité pendant 10 minutes avec un agitateur à pales tournant à 500 tours/minute pour obtenir une solution très homogène.
b. 2,97 g de tensioactif sont ajoutés à la solution obtenue à l'étape a (Cremophor ELP = 5% par rapport à la masse totale de ladite solution) et le mélange est agité pendant 10 minutes avec un agitateur à pales tournant à 500 tours/minute pour obtenir une solution très homogène (et sans bulles de par la présence d'antimousse).
c. La vitesse de rotation des pales de l'agitateur est augmentée à 1000 tours/minutes et 150 mL de la seconde phase liquide (non miscible à la première), cyclohexane ou heptane, sont incorporés très progressivement (habituellement 44 gouttes/minute).
d. L'émulsion obtenue est versée dans un récipient fermant hermétiquement pour éviter l'évaporation des solvants volatils utilisés et mise à l'étuve à 70 °C pendant 24h. La gélation se fait en 40 - 45 minutes, et un bon durcissement du matériau et de meilleures propriétés mécaniques après séchage sont obtenus après 24h.
e. Lorsque la seconde phase liquide est du cyclohexane ou de l'heptane, aucun lavage au Sohxlet n'est nécessaire pour révéler la porosité, contrairement au cas de l'huile. En présence de cyclohexane ou d'heptane, un simple séchage à l'air en 3 - 4 jours suffit pour obtenir un polyHIPE sec et hautement poreux.

Après séchage à l'air pendant 3 - 4 jours un polyHIPE sec et hautement poreux est obtenu.

Un monolithe cellulaire polyHIPE est préparé de la même manière avec de l'huile de tournesol comme seconde phase liquide.

Les conditions de préparation sont résumées dans le tableau ci-dessous pour deux matériaux préparés dans des conditions strictement identiques selon le protocole décrit ci-dessus, à cette différence près que le premier (TC) a été préparé avec du cyclohexane comme seconde phase liquide, et le second (TH) à titre de comparaison avec de l'huile de tournesol comme seconde phase liquide.

| Echantillon | TC | TH |
|---|---|---|
| Fraction massique de solides (%) | 40 | 40 |
| Fraction massique de tensioactif (%) | 5 | 5 |
| Fraction volumique de seconde phase (%) | 75 | 75 |
| pH initial | 2,5 | 2,5 |
| Vitesse de rotation de l'étape b | 500 | 500 |
| Vitesse de rotation de l'étape c | 1000 | 1000 |

### 5.2. Résultats

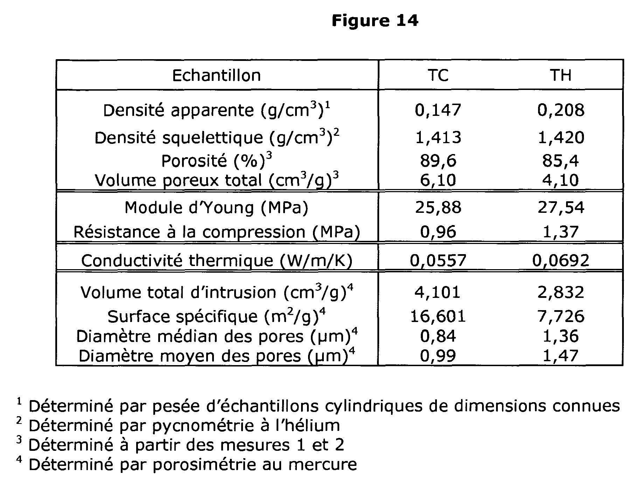

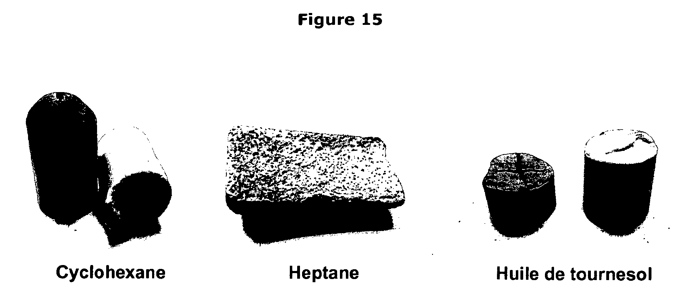

Ils sont donnés dans les figures 14 et 15.

Les propriétés de deux matériaux ainsi préparés (TC) et (TH) sont donnés dans le tableau de la figure 14 et dans la figure 15.

Les matériaux préparés avec le solvant volatil en lieu et place de la phase huileuse sont moins longs et donc plus simples à préparer, et présentent une porosité à la fois plus développée et plus étroite. Ce sont d'excellents isolants thermiques. Les propriétés mécaniques ne sont pas sensiblement différentes de celles de leurs homologues préparés avec de l'huile.

Avec l'heptane comme seconde phase, on obtient un matériau avec des propriétés très proches (figure 15).

### Exemple 6: Procédé de préparation de monolithes cellulaires de type mousse utilisant comme seconde phase de l'air et différents types de tensioactifs

### 6.1. Mode opératoire

La préparation est réalisée selon la méthode décrite dans l'exemple 3 avec un rapport tannin/(eau + tannin) = 40% en masse. La seule différence vient de l'utilisation d'hexaméthylènetétramine (ou hexamine ou HMT) en poudre plutôt qu'en solution.

Différents tensioactifs sont utilisés : Tween 80 (Polyoxyethylene (20) sorbitan monooleate), Pluronic P-123 (Poly(ethylene glycol)-block-poly(propylene glycol)-block-poly(ethylene glycol)) et Pluronic 6800 (Ethylene glycol propylene glycol adipate).

Les conditions de la préparation de la formulation utilisant le Tween 80 sont données ci-dessous :

| | | |
|---|---|---|
| Solution de tannin | Tannin (g) | 20 |
| | Eau (g) | 30 |
| | tannin/(eau + tannin) (%) | 40 |
| | pTSA (g) | 1,12 |
| | Tensioactif (g) | 3 |
| | Tensioactif/ (eau +tannin) (%) | 6 |
| pH initial de la solution de tannin | | 2,8 |
| Temps d'agitation (min) | | 40 |
| Vitesse de rotation (t/min) | | 2000 |

Pour les autres formulations, les conditions de préparation sont identiques.

### 6.2. Résultats

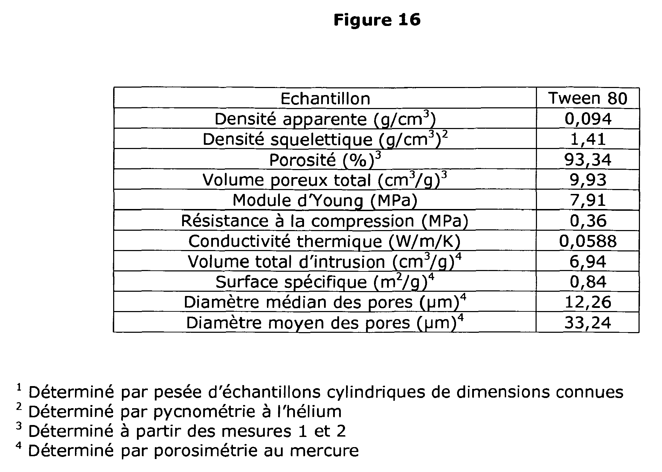

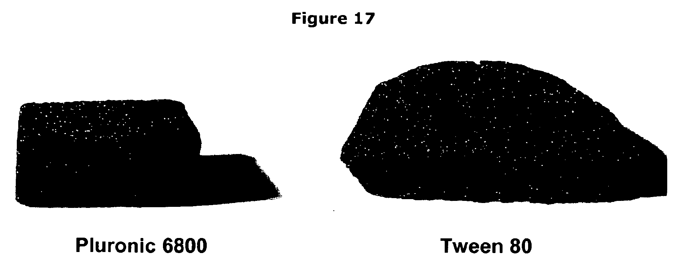

Ils sont donnés dans les figures 16 et 17.

Les propriétés du matériau obtenu à partir de la formulation utilisant du Tween 80 sont directement comparables à celle du matériau F40-40 de l'exemple 3 illustrées à la figure 8, qui a été préparé à partir d'une formulation dans laquelle le tensioactif est du Cremophor ELP (figure 16). Comparé à celui préparé avec du Cremophor ELP, le matériau obtenu à partir de la formulation utilisant du Tween 80 apparaît plus homogène et présente visiblement des cellules de plus petites tailles. Cette caractéristique explique qu'à porosité équivalente, le matériau préparé avec du Tween 80 présente des propriétés mécaniques bien meilleures que celles de l'échantillon F40-40.

Le matériau obtenu à partir de la formulation utilisant du Pluronic 6800 présente également de très bonnes propriétés macroscopiques (figure 17).

### Exemple 7 : Procédé de préparation de monolithes cellulaires polyHIPEs, utilisant comme seconde phase de l'huile végétale, et différentes quantités de durcisseur en poudre.

### 7.1 Mode opératoire

Le but étant d'examiner l'influence de la quantité de durcisseur (hexaméthylènetétramine : HMT) sur les caractéristiques des produits finaux, le protocole de préparation est identique à celui de l'exemple 5 aux exceptions suivantes près :
- les quantités d'eau et de tensioactif sont légèrement modifiées de manière à maintenir constantes les fractions massiques de solides et de tensioactif à 40% et à 5%, respectivement ;
- il n'y a pas d'antimousse ajouté et, pour cette raison, la vitesse d'agitation des pales de l'agitateur est limitée à 250 tours/minute pendant tout le procédé.

Les formulations utilisant différentes quantités de HMT sont alors les suivantes :

| Nom du matériau | TH07 | TH14 | TH19 | TH24 | TH29 |
|---|---|---|---|---|---|
| Tannin (g) | 20 | 20 | 20 | 20 | 20 |
| Eau (g) | 32,1 | 33,17 | 33,9 | 34,65 | 35,4 |
| pTSA (g) | 0,7 | 0,7 | 0,7 | 0,7 | 0,7 |
| HMT (g) | 0,7 | 1,4 | 1,9 | 2,4 | 2,9 |
| Cremophor ELP (g) | 2,82 | 2,9 | 2,97 | 3,04 | 3,11 |
| Huile de tournesol (mL) | 150 | 150 | 150 | 150 | 150 |

### 7.2 Résultats

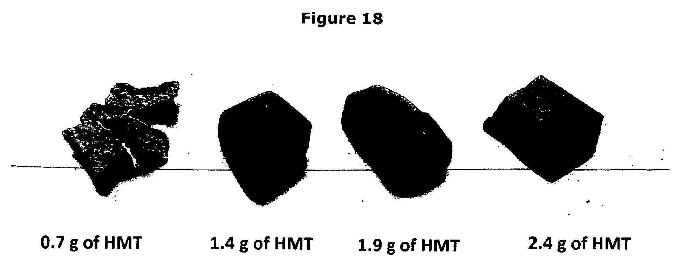

Ils sont donnés dans la figure 18.

L'échantillon TH19 est celui qui présente la structure la plus homogène et les meilleures propriétés macroscopiques.

L'échantillon TH07 est multiplement fissuré et sa structure poreuse est complètement désordonnée et mal définie.

Le matériau TH29 n'a pas pu être préparé car il est impossible d'obtenir l'émulsion initiale en raison d'un durcissement beaucoup trop rapide.

Les échantillons TH14 et TH24 sont assez similaires, mais moins homogènes que TH19, qui est donc le meilleur compromis.

### Exemple 8 : Procédé de préparation de monolithes cellulaires polyHIPEs, utilisant comme seconde phase de l'huile végétale et différents types de tensioactifs

### 8.1. Mode opératoire

Le but étant d'examiner l'influence de la nature du tensioactif sur les caractéristiques des produits finaux, le protocole de préparation est identique à celui de l'exemple 5, aux exceptions suivantes près :
- la quantité de tensioactif introduite à l'étape b est de 1,8 g ;
- dans l'étape c, la vitesse d'agitation est de 1500 tours/minute et la quantité d'huile de tournesol est de 80 mL.

Les différents tensioactifs suivants ont alors été testés : Pluronic 7400 (BASF), Triton X100 (Prolabo), Pluronic 6800 (BASF), TWEEN 80 (Sigma Aldrich), Pluronic 123 (Sigma Aldrich), Pluronic 127 (Sigma Aldrich), Cremophor ELP (Sigma Aldrich).

Les conditions de préparation des différentes formulations sont données dans le tableau ci-dessous :

| Echantillon | Tensioactif |
|---|---|
| Fraction massique de solides (%) | 40 |
| Fraction massique de tensioactif (%) | 3 |
| Fraction volumique de seconde phase (%) | 67 |
| pH initial | 2,5 |
| Vitesse de rotation dans l'étape b | 500 |
| Vitesse de rotation dans l'étape c | 1500 |

### 8.2. Résultats

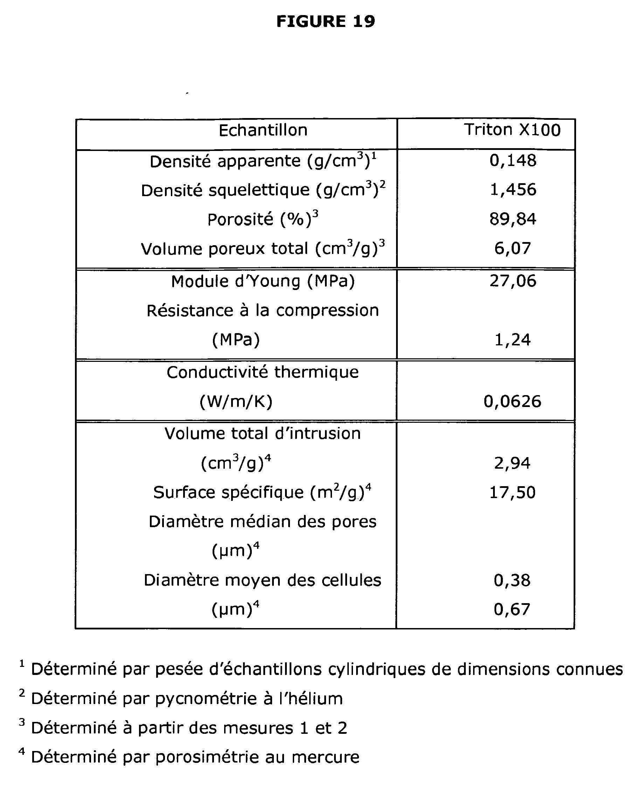

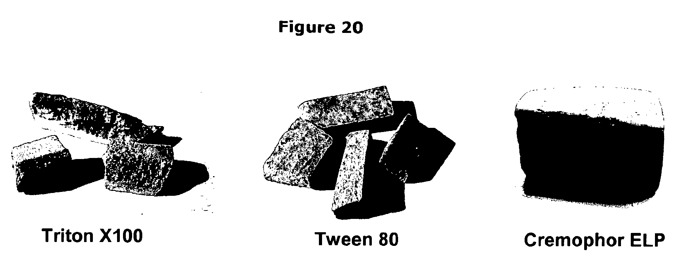

Ils sont donnés dans les figures 19 et 20.

Les observations suivantes peuvent être faites :
i. Pluronic 7400 (BASF): bonne émulsion, de viscosité bien adaptée, difficile à mettre en oeuvre à faible vitesse d'agitation (d'où besoin d'agiter à 1500 tr/min), polyHIPE possible.
ii. Triton X100 (Prolabo): excellente émulsion même à faible vitesse d'agitation, excellent polyHIPE final.
iii. Pluronic 6800 (BASF) : bonne émulsion à condition d'agiter assez vite, bon polyHIPE final.
iv. TWEEN 80 (Sigma Aldrich) : émulsion de viscosité plus faible qu'avec le Cremophor ELP, mais de bonne qualité et homogène même à faible vitesse d'agitation, excellent polyHIPE final.
v. Pluronic 123 (Sigma Aldrich) : belle émulsion, facile à mélanger, bon polyHIPE final.
vi. Pluronic 127 (Sigma Aldrich) : belle émulsion mais seulement après mélange à haute vitesse, long et difficile car Pluronic 127 est peu soluble, bon polyHIPE final.
vii. Cremophor ELP (Sigma Aldrich): parfait dans presque toutes les conditions.

Comparé à son homologue préparé avec du Cremophor ELP, le matériau ayant été préparé à base de Triton X100 est plus poreux, et la porosité est constituée de pore plus fins. Il en résulte des propriétés mécaniques supérieures à celles du matériau préparé avec du Cremophor ELP dans les mêmes conditions.

Le matériau obtenu à partir de la formulation utilisant du Tween 80 présente également de bonnes propriétés macroscopiques (figure 20).

### Exemple 9: Procédé de préparation de monolithes cellulaires polyHIPEs utilisant comme seconde phase de l'huile végétale, un mélange d'HMT et d'alcool furfurylique comme durcisseur et différentes quantités de Cremophor ELP comme tensioactif

### 9.1. Mode opératoire

L'alcool furfurylique a été utilisé comme durcisseur à concentration constante, en plus de l'HMT, et différentes quantités du tensioactif Cremophor ELP ont été utilisées.

Le protocole de préparation est identique à celui de l'exemple 5, aux exceptions suivantes près :
- 5 g d'alcool furfurylique ont été ajoutés en plus des ingrédients déjà décrits dans l'étape a ; l'antimousse n'a pas été ajouté, la présence d'alcool furfurylique limitant visiblement l'aération de la solution pendant le mélange ;
- la quantité de Cremophor ELP a été changée dans la gamme 2 - 10 % par rapport à la masse totale de solution ;
- la température de l'étuve dans l'étape d est de 85°C ;
- dans l'étape e, une étape d'extraction de l'huile au Soxhlet par de l'acétone pendant 7 jours est ajoutée avant l'étape de séchage à température ambiante. A noter que, pendant l'étape de séchage, des fissures peuvent survenir dans les monolithes. Cette fissuration peut être fortement limitée par l'addition dans la formulation de 5 % en masse (par rapport à la masse totale de solution) d'éthylène glycol ou de 5 - 10 % en masse (par rapport à la masse totale de solution) de glycérol.

Les formulations sont alors les suivantes :

| Nom du matériau | TFA2 | TFA4 | TFA6 | TFA8 | TFA10 |
|---|---|---|---|---|---|
| Tannin (g) | 20 | 20 | 20 | 20 | 20 |
| Alcool furfurylique (g) | 5 | 5 | 5 | 5 | 5 |
| Eau (g) | 33,9 | 33,9 | 33,9 | 33,9 | 33,9 |
| pTSA (g) | 0,7 | 0,7 | 0,7 | 0,7 | 0,7 |
| HMT (g) | 1,9 | 1,9 | 1,9 | 1,9 | 1,9 |
| Cremophor ELP (g) | 1,15 | 2,35 | 3,6 | 4,91 | 6,28 |
| Huile de tournesol (mL) | 150 | 150 | 150 | 150 | 150 |

Pour les deux polyHIPEs obtenus dans les conditions décrites ci-dessus avec 1,15 g de Cremophor ELP, soit 2% en masse, l'un (TFA2) ne contenant pas d'éthylène glycol et l'autre (TFA2EG) en contenant 5% en masse (par rapport à la masse totale de solution), les conditions de formulation sont les suivantes :

| Echantillon | TFA2 | TFA2EG |
|---|---|---|
| Fraction massique de solides (%) | 40 | 40 |
| Fraction massique de tensioactif (%) | 2,0 | 2,0 |
| Fraction volumique de seconde phase (%) | 75 | 75 |
| Fraction massique d'éthylène glycol (%) | 0 | 5 |
| pH initial | 2,5 | 2,5 |
| Vitesse de rotation dans l'étape b | 500 | 500 |
| Vitesse de rotation dans l'étape c | 1000 | 1000 |

### 9.2. Résultats :

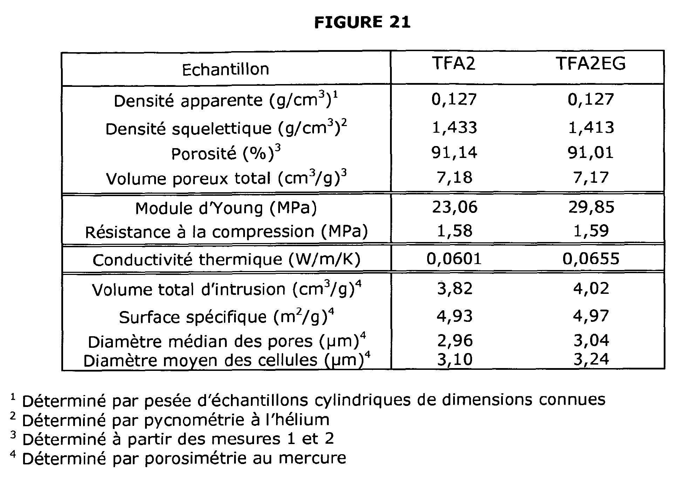

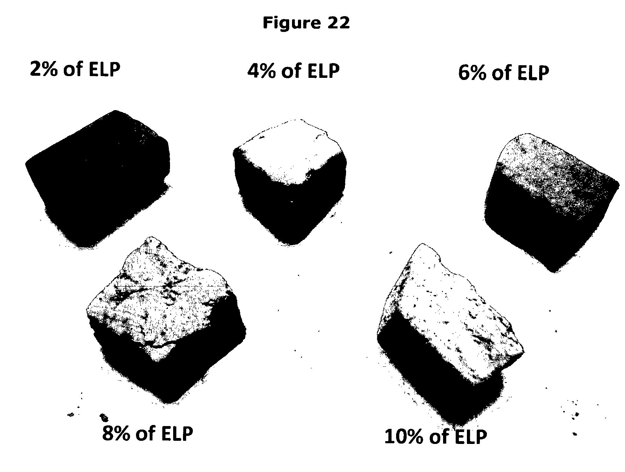

Ils sont donnés dans les figures 21 et 22.

La meilleure gamme de concentrations de tensioactif est 2 - 6% en masse. Les monolithes préparés avec des pourcentages supérieurs (8 et 10%) ont tendance à s'effriter lors de l'étape d'extraction de l'huile en Soxhlet, mais aussi pendant le séchage ultérieur ; ces matériaux sont en effet très friables. Ceux préparés aux plus basses teneurs en tensioactif sont plus stables et plus homogènes.

La présence d'un peu d'éthylène glycol ne change pas significativement les résultats, qu'il s'agisse de la porosité ou des propriétés physiques résultantes. En revanche, la tenue à la fissuration est très bonne.

Le matériau TFA2 peut être comparé au monolithe F75A de l'exemple 1 (voir figures 1 et 13). L'addition de 2% de Cremophor ELP au lieu de 5% donne un matériau à la porosité plus développée, et donc avec des propriétés mécaniques un peu inférieures, et dont les pores sont un peu plus larges.

Les résultats des exemples montrent que :
- tous les pH entre 2 et 8 sont utilisables, donnant des matériaux aux caractéristiques structurales et mécaniques différentes,
- les émulsions eau dans huile comme huile dans eau (on passe des premières aux secondes en augmentant la proportion d'huile, mais sans changer le protocole de préparation) donnent des matériaux monolithiques poreux aux structures poreuses différentes,
- tous les autres paramètres de la formulation, sans exception, jouent un rôle dans l'ajustement de la porosité en termes de volumes de pores, de diamètres de cellules et de connexions entre cellules, de connectivité de la porosité, et de propriétés physiques résultantes, La nature du tensioactif a de ce point de vue un effet particulièrement significatif,
- ainsi, sur la base des procédés décrits, tout type de structure poreuse est désormais accessible, ce qui est nouveau pour des matériaux dérivés de tannins : depuis des structures réticulées jusqu'à des structures cellulaires, en passant par des empilements de sphères creuses connectées,
- les gammes de densités apparentes (et donc de porosités) accessibles sont les plus grandes jamais atteintes pour des monolithes poreux dérivés de tannins,
- les matériaux dérivés, notamment en carbone, conservent la même structure et ont une densité voisine, tout en présentant une porosité plus étroite et des propriétés mécaniques supérieures à celles de leurs homologues organiques.

## Revendications

1. Procédé de production de matériaux monolithiques poreux à base de tannins condensés, ledit procédé comprenant les étapes suivantes :
a. obtenir une première phase liquide, ladite phase liquide étant une solution aqueuse de tannins condensés,
b. obtenir une seconde phase, ladite seconde phase étant soit une huile ou un solvant volatil non miscible à l'eau, soit de l'air, soit un mélange d'huile et d'air ou d'un solvant volatil non miscible à l'eau et d'air et ladite seconde phase n'étant pas miscible dans la première phase, et l'une au moins desdites phases comprenant un tensioactif,
c. disperser ladite seconde phase dans ladite première phase liquide, en présence d'un durcisseur,
d. mélanger lesdites phases sous agitation jusqu'à obtention :
i. d'une émulsion homogène et stable lorsque la seconde phase est une huile ou un solvant volatil non miscible à l'eau, ou
ii. d'un mélange macroscopiquement homogène mais intermédiaire entre une émulsion et une mousse lorsque la seconde phase est un mélange d'huile et d'air ou d'un solvant volatil non miscible à l'eau et d'air, ou
i. d'une mousse lorsque la seconde phase est de l'air, et
e. soit,
i. réaliser la polymérisation de l'émulsion ou de l'intermédiaire émulsion-mousse obtenus à l'étape d.i. ou à l'étape d.ii jusqu'à l'obtention d'un solide, laver si nécessaire et sécher ledit solide, soit,
ii. réaliser la polymérisation et sécher ladite mousse obtenue à l'étape d.iii.

2. Procédé de production de matériaux monolithiques poreux selon la revendication 1 **caractérisé en ce que** :
A. soit la première phase liquide est une phase aqueuse de tannin condensé contenant éventuellement un agent antimousse et la seconde phase une huile végétale ou un solvant volatil non miscible à l'eau et on obtient, après durcissement de la phase aqueuse, extraction de l'huile lorsque la seconde phase est de l'huile végétale, puis séchage, un polyHIPE,
B. soit la première phase liquide est une phase aqueuse de tannin condensé contenant éventuellement un agent antimousse et la seconde phase de l'air et on obtient, après durcissement de la phase aqueuse puis séchage, une mousse rigide,
C. soit la première phase liquide est une phase aqueuse de tannin condensé et la seconde phase un mélange d'huile végétale et d'air ou d'un solvant volatil non miscible à l'eau et d'air et on obtient, après durcissement de la phase aqueuse, extraction de l'huile lorsque la seconde phase est de l'huile végétale, puis séchage, un matériau aéré « de type polyHIPE ».

3. Procédé de production de matériaux monolithiques poreux selon l'une quelconque des revendications précédentes **caractérisé en ce que** la concentration en tannin condensé dans la solution aqueuse de tannin condensé est comprise entre 20 et 60 % en masse de la masse totale (tannin condensé + eau) et est avantageusement égale à 40 % en masse de la masse totale (tannin condensé + eau).

4. Procédé de production de matériaux monolithiques poreux selon l'une quelconque des revendications précédentes **caractérisé en ce que** le pH de la solution de tannin condensé est compris entre 2 et 8.

5. Procédé de production de matériaux monolithiques poreux selon la revendication 2 **caractérisé en ce que** lorsque la première phase liquide est une phase aqueuse de tannin condensé et la seconde phase une huile végétale ou un solvant volatil non miscible à l'eau, le rapport huile/solution aqueuse de tannin condensé ou solvant volatil/solution aqueuse de tannin condensé est compris entre 0,4/1 et 4/1 en volume, et lorsque la première phase liquide est une phase aqueuse de tannin condensé et la seconde phase est un mélange d'huile végétale et d'air ou de solvant volatil non miscible à l'eau et d'air, le rapport huile/solution aqueuse de tannin condensé ou solvant volatil non miscible à l'eau /solution aqueuse de tannin condensé est compris entre 0,3/1 et 4/1 en volume.

6. Procédé de production de matériaux monolithiques poreux selon l'une quelconque des revendications précédentes **caractérisé en ce que** le tannin condensé est choisi dans le groupe comprenant les tannins de type prodelphinidine, de type procyanidine, de type prorobinetinidine, de type profisetinidine, et toute combinaison desdits quatre types de tannin en toutes proportions et est avantageusement du tannin de mimosa, de pin ou de quebracho et/ou le tensioactif est choisi parmi les tensioactifs non ioniques.

7. Procédé de production de matériaux monolithiques poreux selon l'une quelconque des revendications précédentes **caractérisé en ce que** le durcisseur est choisi dans le groupe comprenant les aldéhydes ou les composés susceptibles de se décomposer en aldéhydes, les oxazolidines, les nitroparaffines, l'alcool furfurylique, ainsi que toute combinaison de ces durcisseurs entre eux en toutes proportions.

8. Procédé de production de matériaux monolithiques poreux polyHIPE ou de type polyHIPE selon l'une quelconque des revendications 2 à 7 dans lequel la première phase liquide est une phase aqueuse de tannin condensé et la seconde phase une huile végétale ou un mélange d'huile végétale et d'air **caractérisé en ce qu'**il comprend les étapes suivantes :
a. préparer une solution de tannin condensé dans l'eau, et ajouter éventuellement un agent antimousse dans le cas des polyHIPES,
b. ajuster le pH de la solution obtenue à l'étape a à une valeur comprise entre 2 et 8,
c. agiter la solution obtenue à l'étape précédente à une vitesse comprise entre 200 et 2000 t/min jusqu'à obtention d'une solution homogène,
d. ajouter le tensioactif et maintenir sous agitation à une vitesse comprise entre 200 et 2000 t/min jusqu'à obtention d'une solution homogène,
e. incorporer goutte à goutte l'huile végétale à la solution obtenue à l'étape d, en maintenant l'agitation du mélange à une vitesse comprise entre 200 et 2000 t/min jusqu'à obtention d'une émulsion stable et homogène,
f. incorporer le durcisseur à mi-durée de l'étape e et poursuivre l'agitation,
g. réaliser la polymérisation à une température supérieure à la température ambiante, avantageusement comprise entre 40 et 90 °C, encore plus avantageusement égale à 85 °C, jusqu'à l'obtention d'une émulsion solide,
h. laver le solide obtenu à l'étape précédente avec un solvant organique,
i. sécher le monolithe polyHIPE ou de type polyHIPE.

9. Procédé de production de matériaux monolithiques poreux polyHIPE ou de type polyHIPE selon la revendication 8 **caractérisé en ce que** l'huile végétale de la seconde phase, seule ou en mélange avec l'air, est remplacée par un solvant volatil non miscible à l'eau et l'étape de lavage h) est supprimée.

10. Procédé de production de matériaux monolithiques poreux polyHIPE ou de type polyHIPE selon la revendication 8 ou la revendication 9 **caractérisé en ce que** le tensioactif au lieu d'être ajouté à la solution de tannin condensé est ajouté à l'huile ou au solvant volatil non miscible à l'eau.

11. Procédé de production de matériaux monolithiques poreux polyHIPE ou de type polyHIPE selon l'une quelconque des revendications 2 à 10 comprenant les étapes suivantes :
a. préparer un mélange contenant de l'eau, de l'huile végétale, un durcisseur, et un tensioactif et éventuellement un agent antimousse dans le cas des poyHIPES,
b. ajuster le pH du mélange obtenu à l'étape a, à une valeur comprise entre 2 et 8,
c. agiter le mélange obtenu à l'étape précédente à une vitesse comprise entre 200 et 2000 t/min jusqu'à obtention d'une émulsion stable et homogène,
d. ajouter le tannin condensé en poudre à l'émulsion obtenue à l'étape c et agiter entre 200 et 2000 t/min jusqu'à obtention d'une nouvelle émulsion stable et homogène,
e. réaliser la polymérisation à une température supérieure à la température ambiante, avantageusement comprise entre à 40 et 90 °C, encore plus avantageusement égale à 85 °C, jusqu'à l'obtention d'une émulsion solide,
f. laver le solide obtenu à l'étape précédente avec un solvant organique apte à dissoudre et extraire l'huile,
g. sécher le monolithe rendu hautement poreux.

12. Procédé de production de matériaux monolithiques poreux polyHIPE ou de type polyHIPE selon la revendication 11 **caractérisé en ce que** l'huile végétale est remplacée par un solvant volatil non miscible à l'eau et l'étape de lavage est supprimée.

13. Procédé de production de matériaux monolithiques poreux dans lequel la première phase liquide est une phase aqueuse de tannin condensé et la seconde phase de l'air, **caractérisé en ce qu'**il comprend les étapes suivantes :
a. préparer une solution de tannin condensé dans l'eau,
b. ajuster le pH de la solution obtenue à l'étape a, à une valeur comprise entre 2 et 8,
c. agiter la solution obtenue à l'étape précédente à une vitesse comprise entre 400 et 600 t/min jusqu'à obtention d'une solution homogène,
d. ajouter le tensioactif à la solution obtenue à l'étape c et maintenir sous agitation à une vitesse comprise entre 200 et 2000 t/min jusqu'à l'obtention d'une mousse liquide homogène,
e. incorporer le durcisseur à mi-durée de l'étape d et poursuivre l'agitation,
f. réaliser la polymérisation à une température supérieure à la température ambiante, avantageusement comprise entre à 40 et 90 °C, encore plus avantageusement égale à 85 °C pendant au moins 24 heures,
g. sécher le monolithe pour obtenir une mousse rigide.

14. Matériau monolithique poreux polyHIPE, de type polyHIPE ou sous forme de mousse rigide, susceptible d'être obtenu par polymérisation de tannins condensés par un procédé selon l'une quelconque des revendications 1 à 13.

15. Emulsion utile pour la préparation d'un matériau polyHIPE ou de type polyHIPE selon la revendication 14, **caractérisée en ce qu'**elle comprend une première phase qui est une solution aqueuse de tannins condensés contenant un durcisseur, et une seconde phase à base d'huile notamment d'huile végétale à base de solvant volatil non miscible à l'eau ou à base d'un mélange d'huile et d'air, notamment d'huile végétale et d'air, ou à base d'un mélange d'un solvant volatil non miscible à l'eau et d'air, et l'une au moins desdites phases comprenant un tensioactif.

16. Mousse liquide utile pour la préparation d'une mousse rigide selon la revendication 14, **caractérisée en ce qu'**elle comprend une première phase qui est une solution aqueuse de tannins condensés contenant un tensioactif et un durcisseur et une seconde phase qui est de l'air.

17. Monolithe choisi dans le groupe comprenant un monolithe organique poreux fonctionnel, notamment doté de propriétés catalytiques, susceptible d'être obtenu par fonctionnalisation d'un matériau monolithique poreux selon la revendication 14, un monolithe carboné poreux susceptible d'être obtenu par pyrolyse d'un matériau monolithique poreux selon la revendication 14 et un monolithe poreux en carbure de silicium (SiC) ou composite Si - SiC susceptible d'être obtenu :
a. soit par imprégnation par des polymères précéramiques puis pyrolyse d'un matériau monolithique poreux selon la revendication 14,
b. soit par infiltration de silicium liquide ou gazeux d'un monolithe carboné poreux,
c. soit par combinaison des deux méthodes.

## Patentansprüche

1. Verfahren zur Herstellung von porösen, monolithischen Materialien auf der Basis von kondensierten Tanninen, wobei das Verfahren die folgenden Schritte umfasst:
a. Erhalten einer ersten flüssigen Phase, wobei die flüssige Phase eine wässrige Lösung von kondensierten Tanninen ist,
b. Erhalten einer zweiten Phase, wobei die zweite Phase entweder ein Öl oder ein mit Wasser nicht mischbares, flüchtiges Lösungsmittel oder Luft oder ein Gemisch aus Öl und Luft oder aus einem mit Wasser nicht mischbaren, flüchtigen Lösungsmittel und Luft ist, und wobei die zweite Phase in der ersten Phase nicht mischbar ist, und wobei wenigstens eine der Phasen ein Tensid umfasst,
c. Dispergieren der zweiten Phase in der ersten flüssigen Phase in Gegenwart eines Härters,
d. Mischen der Phasen unter Rühren bis zum Erhalt:
i. einer homogenen und stabilen Emulsion, wenn die zweite Phase ein Öl oder ein mit Wasser nicht mischbares, flüchtiges Lösungsmittel ist, oder
ii. einer makroskopisch homogenen Mischung, aber Zwischenmischung zwischen einer Emulsion und einem Schaum, wenn die zweite Phase ein Gemisch aus Öl und Luft oder aus einem mit Wasser nicht mischbaren, flüchtigen Lösungsmittel und Luft ist, oder
i. eines Schaums, wenn die zweite Phase Luft ist, und
e. entweder
i. Durchführen der Polymerisation der Emulsion oder des Emulsion-Schaum-Zwischenprodukts, die in Schritt d.i. oder in Schritt d.ii erhalten werden, bis zum Erhalt eines Feststoffs, falls erforderlich Waschen und Trocknen des Feststoffs, oder
ii. Durchführen der Polymerisation und Trocknen des in Schritt d.iii erhaltenen Schaums.

2. Verfahren zur Herstellung von porösen, monolithischen Materialien nach Anspruch 1, **dadurch gekennzeichnet, dass**:
A. entweder die erste flüssige Phase eine wässrige Phase von kondensiertem Tannin, eventuell mit einem Antischaummittel, und die zweite Phase ein pflanzliches Öl oder ein mit Wasser nicht mischbares, flüchtiges Lösungsmittel ist, und nach Härten der wässrigen Phase, Extraktion des Öls, wenn die zweite Phase pflanzliches Öl ist, dann Trocknen, ein polyHIPE erhalten wird,
B. oder die erste flüssige Phase eine wässrige Phase von kondensiertem Tannin, eventuell mit einem Antischaummittel, und die zweite Phase Luft ist, und nach Härten der wässrigen Phase, dann Trocknen, ein starrer Schaum erhalten wird,
C. oder die erste flüssige Phase eine wässrige Phase von kondensiertem Tannin und die zweite Phase ein Gemisch aus pflanzlichem Öl und Luft oder aus einem mit Wasser nicht mischbaren, flüchtigen Lösungsmittel und Luft ist, und nach Härten der wässrigen Phase, Extraktion des Öls, wenn die zweite Phase pflanzliches Öl ist, dann Trocknen, ein belüftetes Material "vom Typ polyHIPE" erhalten wird.

3. Verfahren zur Herstellung von porösen, monolithischen Materialien nach einem der vorhergehenden Ansprüche, **dadurch gekennzeichnet, dass** die Konzentration an kondensiertem Tannin in der wässrigen Lösung von kondensiertem Tannin zwischen 20 und 60 Masse-% der Gesamtmasse (kondensiertes Tannin + Wasser) liegt und vorteilhafterweise gleich 40 Masse-% der Gesamtmasse (kondensiertes Tannin + Wasser) beträgt.

4. Verfahren zur Herstellung von porösen, monolithischen Materialien nach einem der vorhergehenden Ansprüche, **dadurch gekennzeichnet, dass** der pH-Wert der Lösung von kondensiertem Tannin zwischen 2 und 8 liegt.

5. Verfahren zur Herstellung von porösen, monolithischen Materialien nach Anspruch 2, **dadurch gekennzeichnet, dass** wenn die erste flüssige Phase eine wässrige Phase von kondensiertem Tannin und die zweite Phase ein pflanzliches Öl oder ein mit Wasser nicht mischbares, flüchtiges Lösungsmittel ist, das Verhältnis von Öl/wässrige Lösung von kondensiertem Tannin oder von flüchtigem Lösungsmittel/wässrige Lösung von kondensiertem Tannin zwischen 0,4/1 und 4/1 nach Volumen beträgt, und wenn die erste flüssige Phase eine wässrige Phase von kondensiertem Tannin und die zweite Phase ein Gemisch aus pflanzlichem Öl und Luft oder aus mit Wasser nicht mischbarem, flüchtigem Lösungsmittel und Luft ist, das Verhältnis von Öl/wässriger Lösung von kondensiertem Tannin oder von mit Wasser nicht mischbarem, flüchtigem Lösungsmittel/wässriger Lösung von kondensiertem Tannin zwischen 0,3/1 und 4/1 nach Volumen beträgt.

6. Verfahren zur Herstellung von porösen, monolithischen Materialien nach einem der vorhergehenden Ansprüche, **dadurch gekennzeichnet, dass** das kondensierte Tannin aus der Gruppe umfassend die Tannine vom Typ Prodelphinidin, vom Typ Procyanidin, vom Typ Prorobinetinidin, vom Typ Profisetinidin sowie jedwede Kombination der vier Tannin-Typen in allen Verhältnissen ausgewählt ist und vorteilhafterweise Mimosen-, Kiefer- oder Quebracho-Tannin ist und/oder das Tensid aus den nichtionischen Tensiden ausgewählt ist.

7. Verfahren zur Herstellung von porösen, monolithischen Materialien nach einem der vorhergehenden Ansprüche, **dadurch gekennzeichnet, dass** der Härter aus der Gruppe umfassend die Aldehyde oder die Verbindungen, die geeignet sind, sich in Aldehyde zu spalten, die Oxazolidine, die Nitroparaffine, Furfurylalkohol sowie jedwede Kombination dieser Härter untereinander in allen Verhältnissen ausgewählt ist.

8. Verfahren zur Herstellung von porösen, monolithischen polyHIPE- oder polyHIPE-artigen Materialien nach einem der Ansprüche 2 bis 7, bei dem die erste flüssige Phase eine wässrige Phase von kondensiertem Tannin und die zweite Phase ein pflanzliches Öl oder ein Gemisch aus pflanzlichem Öl und Luft ist, **dadurch gekennzeichnet, dass** es die folgenden Schritte umfasst:
a. Herstellen einer Lösung von kondensiertem Tannin in Wasser und eventuell Zugeben eines Antischaummittels im Fall der polyHIPEs,
b. Einstellen des pH-Wertes der in Schritt a erhaltenen Lösung auf einen Wert zwischen 2 und 8,
c. Rühren der in dem vorhergehenden Schritt erhaltenen Lösung mit einer Geschwindigkeit zwischen 200 und 2000 U/Min. bis zum Erhalt einer homogenen Lösung,
d. Zugeben des Tensids und Halten unter Rühren mit einer Geschwindigkeit zwischen 200 und 2000 U/Min. bis zum Erhalt einer homogenen Lösung,
e. tropfenweises Beimengen des pflanzlichen Öls zu der in Schritt d erhaltenen Lösung, unter Beibehalten des Rührens der Mischung mit einer Geschwindigkeit zwischen 200 und 2000 U/Min. bis zum Erhalt einer stabilen und homogenen Emulsion,
f. Beimengen des Härters bei halber Dauer des Schrittes e und Fortsetzen des Rührens,
g. Durchführen der Polymerisation bei einer Temperatur oberhalb der Raumtemperatur, vorteilhafterweise zwischen 40 und 90 °C, in noch vorteilhafterer Weise von gleich 85 °C, bis zum Erhalt einer festen Emulsion,
h. Waschen des in dem vorhergehenden Schritt erhaltenen Feststoffs mit einem organischen Lösungsmittel,
i. Trocknen des polyHIPE- oder polyHIPE-artigen Monolithen.

9. Verfahren zur Herstellung von porösen, monolithischen polyHIPE- oder polyHIPE-artigen Materialien nach Anspruch 8, **dadurch gekennzeichnet, dass** das pflanzliche Öl der zweiten Phase, allein oder in Mischung mit Luft, durch ein mit Wasser nicht mischbares, flüchtiges Lösungsmittel ersetzt wird und der Waschschritt h) weggelassen wird.

10. Verfahren zur Herstellung von porösen, monolithischen polyHIPE- oder polyHIPE-artigen Materialien nach Anspruch 8 oder Anspruch 9, **dadurch gekennzeichnet, dass** das Tensid, anstatt der Lösung von kondensiertem Tannin zugegeben zu werden, dem Öl oder dem mit Wasser nicht mischbaren, flüchtigen Lösungsmittel zugegeben wird.

11. Verfahren zur Herstellung von porösen, monolithischen polyHIPE- oder polyHIPE-artigen Materialien nach einem der Ansprüche 2 bis 10, das die folgenden Schritte umfasst:
a. Herstellen einer Mischung, die Wasser, pflanzliches Öl, einen Härter und ein Tensid und eventuell ein Antischaummittel im Fall der polyHIPEs umfasst,
b. Einstellen des pH-Wertes des in Schritt a erhaltenen Gemischs auf einen Wert zwischen 2 und 8,
c. Rühren des in dem vorhergehenden Schritt erhaltenen Gemischs mit einer Geschwindigkeit zwischen 200 und 2000 U/Min. bis zum Erhalt einer stabilen und homogenen Emulsion,
d. Zugeben des kondensierten, pulverförmigen Tannins zu der in Schritt c erhaltenen Emulsion und Rühren zwischen 200 und 2000 U/Min. bis zum Erhalt einer neuen stabilen und homogenen Emulsion,
e. Durchführen der Polymerisation bei einer Temperatur oberhalb der Raumtemperatur, vorteilhafterweise zwischen 40 und 90 °C, in noch vorteilhafterer Weise von gleich 85 °C, bis zum Erhalt einer festen Emulsion,
f. Waschen des in dem vorhergehenden Schritt erhaltenen Feststoffs mit einem organischen Lösungsmittel, das geeignet ist, das Öl zu lösen und zu extrahieren,
g. Trocknen des hochporös gemachten Monolithen.

12. Verfahren zur Herstellung von porösen, monolithischen polyHIPE- oder polyHIPE-artigen Materialien nach Anspruch 11, **dadurch gekennzeichnet, dass** das pflanzliche Öl durch ein mit Wasser nicht mischbares, flüchtiges Lösungsmittel ersetzt wird und der Waschschritt weggelassen wird.

13. Verfahren zur Herstellung von porösen, monolithischen Materialien, bei dem die erste flüssige Phase eine wässrige Phase von kondensiertem Tannin und die zweite Phase Luft ist, **dadurch gekennzeichnet, dass** es die folgenden Schritte umfasst:
a. Herstellen einer Lösung von kondensiertem Tannin in Wasser,
b. Einstellen des pH-Wertes der in Schritt a erhaltenen Lösung auf einen Wert zwischen 2 und 8,
c. Rühren der in dem vorhergehenden Schritt erhaltenen Lösung mit einer Geschwindigkeit zwischen 400 und 600 U/Min. bis zum Erhalt einer homogenen Lösung,
d. Zugeben des Tensids zu der in Schritt c erhaltenen Lösung und Halten unter Rühren mit einer Geschwindigkeit zwischen 200 und 2000 U/Min. bis zum Erhalt eines homogenen, flüssigen Schaums,
e. Beimengen des Härters bei halber Dauer des Schrittes d und Fortsetzen des Rührens,
f. Durchführen der Polymerisation bei einer Temperatur oberhalb der Raumtemperatur, vorteilhafterweise zwischen 40 und 90 °C, in noch vorteilhafterer Weise von gleich 85 °C, für wenigstens 24 Stunden,
g. Trocknen des Monolithen, um einen starren Schaum zu erhalten.

14. Poröses, monolithisches polyHIPE-, polyHIPE-artiges Material oder in Form von starrem Schaum, das geeignet ist, durch Polymerisation von kondensierten Tanninen mittels eines Verfahrens nach einem der Ansprüche 1 bis 13 erhalten zu werden.

15. Emulsion, die für die Herstellung eines polyHIPE- oder polyHIPE-artigen Materials nach Anspruch 14 nützlich ist, **dadurch gekennzeichnet, dass** sie eine erste Phase umfasst, die eine wässrige Lösung von kondensierten Tanninen, welche einen Härter enthält, ist, sowie eine zweite Phase auf der Basis von Öl, insbesondere von pflanzlichem Öl, auf der Basis von mit Wasser nicht mischbarem, flüchtigem Lösungsmittel oder auf der Basis eines Gemischs aus Öl und Luft, insbesondere pflanzlichem Öl und Luft, oder auf der Basis eines Gemischs aus einem mit Wasser nicht mischbaren, flüchtigen Lösungsmittel und Luft, und wobei wenigstens eine der Phasen ein Tensid umfasst.

16. Flüssiger Schaum, der für die Herstellung eines starren Schaums nach Anspruch 14 nützlich ist, **dadurch gekennzeichnet, dass** er eine erste Phase, die eine wässrige Lösung von kondensierten Tanninen, welche ein Tensid und einen Härter enthält, ist, sowie eine zweite Phase, die Luft ist, umfasst.

17. Monolith, ausgewählt aus der Gruppe umfassend einen funktionellen, organischen, porösen Monolithen, der insbesondere mit katalytischen Eigenschaften ausgestattet ist, der geeignet ist, durch Funktionalisieren eines porösen, monolithischen Materials nach Anspruch 14 erhalten zu werden, einen porösen kohlenstoffhaltigen Monolithen, der geeignet ist, durch Pyrolyse eines porösen, monolithischen Materials nach Anspruch 14 erhalten zu werden, und einen porösen Monolithen aus Siliziumkarbid (SiC) oder Si-SiC-Verbundwerkstoff, der geeignet ist:
a. entweder durch Imprägnieren mit präkeramischen Polymeren, dann Pyrolyse eines porösen, monolithischen Materials nach Anspruch 14,
b. oder durch Infiltration eines porösen, kohlenstoffhaltigen Monolithen mit flüssigem oder gasförmigem Silizium,
c. oder durch Kombination der beiden Methoden
erhalten zu werden.

## Claims

1. Process for the production of porous monolithic materials based on condensed tannins, said process comprising the following steps:
a. obtaining a first liquid phase, said liquid phase being an aqueous solution of condensed tannins,
b. obtaining a second phase, said second phase being either an oil or a volatile solvent not miscible with water, or air, or a mixture of oil and air or of a volatile solvent not miscible with water and air and said second phase not being miscible with the first phase, and at least one of said phases comprising a surfactant,
c. dispersing said second phase in said first liquid phase, in the presence of a hardening agent,
d. mixing said phases with stirring until the obtention:
i. of a homogeneous and stable emulsion when the second phase is an oil or a volatile solvent not miscible with water, or
ii. of a mixture which is macroscopically homogeneous but intermediate between an emulsion and a foam when the second phase is a mixture of oil and air or of a volatile solvent not miscible with water and air, or
i. of a foam when the second phase is air, and
e. either,
i. carrying out the polymerization of the emulsion or of the emulsion-foam intermediate obtained in step d.i) or in step d.ii) until the obtention of a solid, washing if necessary and drying said solid, or,
ii. carrying out the polymerization and drying said foam obtained in step d.iii).

2. Process for the production of porous monolithic materials according to claim 1, **characterized in that**:
A. either the first liquid phase is an aqueous phase of condensed tannin optionally containing an antifoaming agent and the second phase a vegetable oil or a volatile solvent not miscible with water and, after hardening of the aqueous phase, extraction of the oil when the second phase is vegetable oil, then drying, a polyHIPE is obtained,
B. or the first liquid phase is an aqueous phase of condensed tannin optionally containing an antifoaming agent and the second phase air and, after hardening of the aqueous phase then drying, a rigid foam is obtained,
C. or the first liquid phase is an aqueous phase of condensed tannin and the second phase a mixture of vegetable oil and air or of a volatile solvent not miscible with water and air and, after hardening of the aqueous phase, extraction of the oil when the second phase is vegetable oil, then drying, an aerated "polyHIPE-type" material is obtained.

3. Process for the production of porous monolithic materials according to any one of the preceding claims, **characterized in that** the concentration of condensed tannin in the aqueous solution of condensed tannin is comprised between 20 and 60% by mass of the total mass (condensed tannin + water) and is advantageously equal to 40% by mass of the total mass (condensed tannin + water).

4. Process for the production of porous monolithic materials according to any one of the preceding claims, **characterized in that** the pH of the solution of condensed tannin is comprised between 2 and 8.

5. Process for the production of porous monolithic materials according to claim 2, **characterized in that** when the first liquid phase is an aqueous phase of condensed tannin and the second phase a vegetable oil or a volatile solvent not miscible with water, the ratio oil/aqueous solution of condensed tannin or volatile solvent/aqueous solution of condensed tannin is comprised between 0.4/1 and 4/1 by volume and when the first liquid phase is an aqueous phase of condensed tannin and the second phase is a mixture of vegetable oil and air or of volatile solvent not miscible with water and air, the ratio oil/aqueous solution of condensed tannin or volatile solvent not miscible with water/aqueous solution of condensed tannin is comprised between 0.3/1 and 4/1 by volume.

6. Process for the production of porous monolithic materials according to any one of the preceding claims, **characterized in that** the condensed tannin is selected from the group comprising tannins of the prodelphinidine type, procyanidine type, prorobinetinidine type or profisetinidine type, and any combination of said four types of tannin in any proportions and advantageously is mimosa, pine or quebracho tannin and/or the surfactant is selected from the non-ionic surfactants.

7. Process for the production of porous monolithic materials according to any one of the preceding claims, **characterized in that** the hardening agent is selected from the group comprising aldehydes or compounds capable of decomposing into aldehydes, oxazolidines, nitroparaffins or furfuryl alcohol, and any combination of these hardening agents with one another in any proportions.

8. The process for production of porous polyHIPE or polyHIPE-type monolithic materials as claimed in any one of claims 2 to 7, wherein the first liquid phase is an aqueous phase of condensed tannin and the second phase a vegetable oil or a mixture of vegetable oil and air **characterized in that** it comprises the following steps:
a. preparing a solution of condensed tannin in water, and if necessary adding an antifoaming agent in the case of the polyHIPEs,
b. adjusting the pH of the solution obtained in step a) to a value between 2 and 8,
c. stirring the solution obtained in the previous step at a speed comprised between 200 and 2000 rpm until the obtention of a homogeneous solution,
d. adding the surfactant and maintaining under stirring at a speed comprised between 200 and 2000 rpm until the obtention of a homogeneous solution,
e. incorporating the vegetable oil dropwise into the solution obtained in step d), while maintaining stirring of the mixture at a speed comprised between 200 and 2000 rpm until the obtention of a stable and homogeneous emulsion,
f. incorporating the hardening agent halfway through step e) and continuing stirring,
g. carrying out the polymerization at a temperature greater than ambient temperature, advantageously comprised between 40 and 90°C, still more advantageously equal to 85°C, until the obtention of a solid emulsion,
h. washing the solid obtained in the previous step with an organic solvent,
i. drying the polyHIPE or polyHIPE-type monolith.

9. Process for the production of porous polyHIPE or polyHIPE-type monolithic materials according to claim 8, **characterized in that** the vegetable oil of the second phase, alone or in a mixture with air, is replaced with a volatile solvent not miscible with water and the washing step h) is eliminated.

10. Process for the production of porous polyHIPE or polyHIPE-type monolithic materials according to claim 8 or claim 9, **characterized in that** instead of being added to the solution of condensed tannin, the surfactant is added to the oil or to the volatile solvent not miscible with water.

11. Process for the production of porous polyHIPE or polyHIPE-type monolithic materials according to any one of claims 2 to 10, comprising the following steps:
a. preparing a mixture containing water, vegetable oil, a hardening agent, and a surfactant and possibly an antifoaming agent in the case of the polyHIPEs,
b. adjusting the pH of the mixture obtained in step a), to a value comprised between 2 and 8,
c. stirring the mixture obtained in the previous step at a speed comprised between 200 and 2000 rpm until the obtention of a stable and homogeneous emulsion,
d. adding the condensed tannin in powder form to the emulsion obtained in step c) and stirring at between 200 and 2000 rpm until the obtention of a new stable and homogeneous emulsion,
e. carrying out the polymerization at a temperature greater than ambient temperature, advantageously comprised between 40 and 90°C, still more advantageously equal to 85°C, until the obtention of a solid emulsion,
f. washing the solid obtained in the previous step with an organic solvent suitable for dissolving and extracting the oil,
g. drying the monolith, which has been rendered highly porous.

12. Process for the production of porous polyHIPE or polyHIPE-type monolithic materials according to claim 11, **characterized in that** the vegetable oil is replaced with a volatile solvent not miscible with water and the washing step is eliminated.

13. Process for the production of porous monolithic materials in which the first liquid phase is an aqueous phase of condensed tannin and the second phase air, **characterized in that** it comprises the following steps:
a. preparing a solution of condensed tannin in water,
b. adjusting the pH of the solution obtained in step a), to a value comprised between 2 and 8,
c. stirring the solution obtained in the previous step at a speed comprised between 400 and 600 rpm until the obtention of a homogeneous solution,
d. adding the surfactant to the solution obtained in step c) and maintaining under stirring at a speed comprised between 200 and 2000 rpm until the obtention of a homogeneous liquid foam,
e. incorporating the hardening agent halfway through step d) and continuing stirring,
f. carrying out the polymerization at a temperature greater than ambient temperature, advantageously comprised between 40 and 90°C, still more advantageously equal to 85°C for at least 24 hours,
g. drying the monolith to obtain a rigid foam.

14. A porous monolithic polyHIPE or polyHIPE-type material or in the form of rigid foam, capable of being obtained by polymerization of condensed tannins by a process according to any one of claims 1 to 13.

15. An emulsion useful for the preparation of a polyHIPE or polyHIPE-type material according to claim 17, **characterized in that** it comprises a first phase which is an aqueous solution of condensed tannins containing a hardening agent, and a second phase based on oil, in particular vegetable oil based on volatile solvent not miscible with water or based on a mixture of oil and air, in particular of vegetable oil and air, or based on a mixture of a volatile solvent not miscible with water and air, and at least one of said phases comprising a surfactant.

16. A liquid foam useful for the preparation of a rigid foam according to claim 14, **characterized in that** it comprises a first phase which is an aqueous solution of condensed tannins containing a surfactant and a hardening agent and a second phase which is air.

17. A monolith selected from the group comprising a functional porous organic monolith, in particular endowed with catalytic properties, capable of being obtained by functionalization of a porous monolithic material according to claim 14, a porous carbonized monolith capable of being obtained by pyrolysis of a porous monolithic material according to claim 14 and a silicon carbide (SiC) or composite Si-SiC porous monolith capable of being obtained:
a. either by impregnation with preceramic polymers then pyrolysis of a porous monolithic material according to claim 14,
b. or by liquid or gaseous silicon infiltration of a porous carbonized monolith,
c. or by combination of the two methods.
